# EUROPÄISCHE PATENTSCHRIFT

(11) **EP 1 153 014 B1**
(45) Veröffentlichungstag und Bekanntmachung des Hinweises auf die Patenterteilung: **22.09.2004**
(21) Anmeldenummer: 00909151.3
(22) Anmeldetag: 08.02.2000
(51) Int. Cl.: C07D 213/74, A61K 31/44, A61K 31/4402, A61P 9/00

(54) **BETA-ALANINDERIVATE**
BETA-ALANINE DERIVATIVES
DERIVES DE LA BETA-ALANINE

(30) Priorität: 20.02.1999 DE 19907370; 01.12.1999 DE 19957787
(43) Veröffentlichungstag der Anmeldung: 14.11.2001
(73) Patentinhaber: MERCK PATENT GmbH, 64293 Darmstadt (DE)
(72) Erfinder: HÖLZEMANN, Günter, D-64342 Seeheim-Jugenheim (DE); GOODMAN, Simon, D-64286 Darmstadt (DE); JONCZYK, Alfred, D-64295 Darmstadt (DE); STÄHLE, Wolfgang, D-55218 Ingelheim (DE)
(86) Internationale Anmeldenummer: PCT/EP2000/000969
(87) Internationale Veröffentlichungsnummer: WO 2000/048996

(56) Entgegenhaltungen:
- WO-A-97/24124
- WO-A-97/26250
- GB-A- 2 327 609

## Beschreibung

Die Erfindung betrifft β-Alaninderivate der Formel I 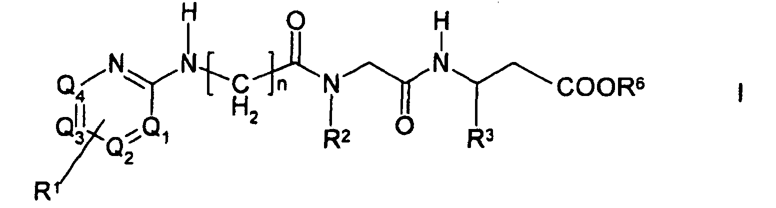 worin
- Q₁, Q₂, Q₃ oder Q₄: jeweils unabhängig voneinander CH oder N,
- R¹: H, A, Ar, Hal, OH, OA, CF₃ oder OCF₃,
- R²: H oder A,
- R³: 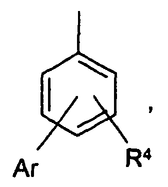
- R⁴: H, A, Hal, OH, OA, CF₃, OCF₃, CN, NH₂, NHA, NA₂ oder NH-C(O)A,
- R⁶: H, A, -(CH₂)ₘ-OH, -(CH₂)ₘ-O-C(O)A oder -(CH₂)ₘ-Ar,
- A: Alkyl mit 1 bis 6 C-Atomen,
- Ar: unsubstituiertes oder ein-, zwei- oder dreifach durch A, CF₃, OH, OA, OCF₃, CN, NO₂ oder Hal substituiertes Aryl, wobei Aryl Phenyl, Naphthyl, Anthryl oder Biphenylyl bedeutet,
- Hal: F, Cl, Br oder I,
- n: 2, 3, 4, 5 oder 6,
- m: 1, 2, 3 oder 4,
bedeutet sowie ihre physiologisch unbedenklichen Salze und Solvate.

Teilweise ähnliche Verbindungen sind aus WO 97/26250 oder WO 97/24124 bekannt.

Der Erfindung lag die Aufgabe zugrunde, neue Verbindungen mit wertvollen Eigenschaften aufzufinden, insbesondere solche, die zur Herstellung von Arzneimitteln verwendet werden.

Es wurde gefunden, daß die Verbindungen der Formel I und ihre Salze bei guter Verträglichkeit sehr wertvolle pharmakologische Eigenschaften besitzen. Vor allem wirken sie als Integrin-inhibitoren, wobei sie insbesondere die Wechselwirkungen der αvβ3- oder αvβ5-Integrin-Rezeptoren mit Liganden hemmen, wie z.B. die Bindung von Vitronectin an den αvβ3-Integrinrezeptor. Integrine sind membrangebundene, heterodimere Glycoproteine, die aus einer α-Untereinheit und einer kleineren β-Untereinheit bestehen. Die relative Affinität und Spezifität für eine Ligandenbindung wird durch Kombination der verschiedenen α- und β-Untereinheiten bestimmt. Besondere Wirksamkeit zeigen die erfindungsgemäßen Verbindungen im Fall der Integrine αvβ1, αvβ3, αvβ5, αIIbβ3 sowie αvβ6 und αvβ8, bevorzugt von αvβ3 und αvβ5. Es wurden insbesondere potente selektive Inhibitoren des Integrins αvβ3 gefunden. Das αvβ3 Integrin wird auf einer Reihe von Zellen, z.B. Endothelzellen, Zellen der glatten Gefäßmuskulatur beispielsweise der Aorta, Zellen zum Abbau von Knochenmatrix (Osteoclasten) oder Tumorzellen, exprimiert.

Die Wirkung der erfindungsgemäßen Verbindungen kann z.B. nach der Methode nachgewiesen werden, die von J.W. Smith et al. in J. Biol. Chem. **1990**, *265,* 12267-12271 beschrieben wird.
Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science **1994**, *264*, 569-571 beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T. Hu, G. Klier und D.A. Cheresh in Cell **1994**, *79*, 1157-1164 beschrieben. Es wurden darin z.B. αvβ3-Antagonisten oder Antikörper gegen αvβ3 beschrieben, die eine Schrumpfung von Tumoren durch Einleiten von Apoptose bewirken.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, analog der Methode von F. Mitjans et al., J. Cell Science **1995**, *108*, 2825-2838.

Die Verbindungen der Formel I können die Bindung von Metalloproteinasen an Integrine hemmen und so verhindern, daß die Zellen die enzymatische Aktivität der Proteinase nutzen können. Ein Beispiel ist in der Hemmbarkeit der Bindung von MMP-2- (Matrix-Metallo-Proteinase-2-) an den Vitronektin-Rezeptor αᵥβ₃ durch ein Cyclo-RGD-Peptid zu finden, wie in P.C. Brooks et al., Cell **1996**, *85*, 683-693 beschrieben.

Verbindungen der Formel I, die die Wechselwirkung von Integrinrezeptoren und Liganden, wie z.B. von Fibrinogen an den Fibrinogenrezeptor (Glycoprotein IIb/IIIa) blockieren, verhindern als Antagonisten die Ausbreitung von Tumorzellen durch Metastase und können daher als antimetastatisch wirkende Substanzen bei Operationen eingesetzt werden, bei denen Tumore chirurgisch entfernt oder angegriffen werden. Dies wird durch folgende Beobachtungen belegt:

Die Verbreitung von Tumorzellen von einem lokalen Tumor in das vaskuläre System erfolgt durch die Bildung von Mikroaggregaten (Mikrothromben) durch die Wechselwirkung der Tumorzellen mit Blutplättchen. Die Tumorzellen sind durch den Schutz im Mikroaggregat abgeschirmt und werden von den Zellen des Immunsystems nicht erkannt. Die Mikroaggregate können sich an Gefäßwandungen festsetzen, wodurch ein weiteres Eindringen von Tumorzellen in das Gewebe erleichtert wird. Da die Bildung der Mikrothromben durch Ligandenbindung an die entsprechenden Integrinrezeptoren, z.B. αvβ3 oder αIIbβ3, auf aktivierten Blutplättchen vermittelt wird, können die entsprechenden Antagonisten als wirksame Metastase-Hemmer angesehen werden.

Die Verbindungen der Formel I können als Arzneimittelwirkstoffe in der Human- und Veterinärmedizin eingesetzt werden, insbesondere zur Prophylaxe und/oder Therapie von Erkrankungen des Kreislaufs, Thrombose, Herzinfarkt, Arteriosklerose, Apoplexie, Angina pectoris, Tumorerkrankungen, wie Tumorentwicklung oder Tumormetastasierung, osteolytischen Krankheiten wie Osteoporose, pathologisch angiogenen Krankheiten wie z.B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, Multiplesklerose, viraler Infektion, bakterieller Infektion, Pilzinfektion, bei akutem Nierenversagen und bei der Wundheilung zur Unterstützung des Heilungsprozesses.

Die Verbindungen der Formel I können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P. Valentin-Weigund et al. in Infection and Immunity, **1988**, 2851-2855 beschriebene Verfahren nachgewiesen werden.

Ausgewählte Verbindungen der Formel I sind insbesondere selektive αvβ3- und αvβ6-Integrinrezeptorinhibitoren.
Ausgewählte Verbindungen der Formel I sind insbesondere selektive αvβ5- und αvβ6-Integrinrezeptorinhibitoren.
Ausgewählte Verbindungen der Formel I sind insbesondere selektive αvβ3- und αvβ5- und αvβ6-Integrinrezeptorinhibitoren.

Die Wirkung einer Verbindung auf einen αvβ5-Integrinrezeptor und damit die Aktivität als Inhibitor kann z.B. nach der Methode nachgewiesen werden, die von J.W. Smith et al. in J. Biol. Chem. **1990**, *265,* 12267-12271 beschrieben wird.
Die Wirkung einer Verbindung auf einen αvβ6-Integrinrezeptor und damit die Aktivität als Inhibitor kann z.B. nach der Methode nachgewiesen werden, die von J.W. Smith et al. in J. Biol. Chem. **1990**, *265*, 12267-12271 beschrieben wird.

Diese ausgewählten Verbindungen eignen sich in besonderer Weise für eine Therapie oder für die Bekämpfung pathologischer Vorgänge, die durch die Integrine αvβ3- und/oder αvβ5- und αvβ6 beeinflußbar sind. Angiogene Pathologien sind beispielsweise Hauterkrankungen wie Psoriasis, bullöser Pemphigus, Dermatitis und Erytheme sowie auch Lungenfibrose, zystische Fibrose, Endometriose, Leberzirrhose oder Periodontitis und werden über αvβ3 und/oder αvβ5 Inhibitoren beeinflußbar, wobei insbesondere Carcinome sowie die oben erwähnten Hauterkrankungen und Lungenfibrose auch Pathologien von Epithelzellen sind, die über αvβ6-Inhibitoren beeinflußbar werden. (Lit.: Healy D.L. et al., Hum. Reprod. Update 1998, 4(5), 736-40; Creamer D. et al., Br. J. Dermatol. 1997, 137, 851-5; Norrby K., APMIS. 1997, 105, 417-37; Creamer D. et al., Br. J. Dermatol. 1997, 136, 859-65; Polverini P.J., Crit. Rev. Oral. Biol. Med. 1995, 6, 230-47; Brown L.F. et al., J. Invest. Dermatol. 1995, 104, 744-9; Hoyt D.G. et al., Am. J. Physiol. 1997, 273, L612-7; Pilewski J.M. et al., ibid 1997, 273, L256-63 oder Goldman M. et al., Gene. Ther. 1996, 3, 811-8).
Eine bevorzugte Anwendung dieser ausgewählten Verbindungen liegt in der Krebstherapie.

Lösungsansätze für eine Krebstherapie konzentrieren sich in der Regel auf die Bekämpfung eines Teilgebietes, eines Kompartiments, in der Entwicklung eines soliden Tumors. Kompartimente sind unter anderem die Entwicklung des Tumors an sich oder die in den Tumor einwachsenden Blutgefäße, die für die Nahrungszufuhr des Tumors verantwortlich sind. Sobald die Nahrungszufuhr des Tumors eingeschränkt wird, lösen transkriptionale Aktivatoren, zumeist über hypoxische oder hypoglykämische Promotoren, die Produktion und Sekretion von polypeptidischen Wachstumsfaktoren aus. Diese aktivieren das Aussprießen von Blutgefäßen. Damit die Endothelzellen des Blutgefäßes sich teilen können sind anti-apoptotische Signale nötig, die von Zelloberflächen-Rezeptoren der Integrinfamilie freigesetzt werden. Insbesondere sind die Integrinrezeptoren αvβ3 und αvβ5 für diese Freisetzung verantwortlich (Lit.: P.C. Brooks, Eur. J. Cancer, 1996, 32A, 2423-2429; P.C. Brooks at al., Cell, 1994, 79, 1157-1164).

Eine Inhibierung dieser Integrinrezeptoren αvβ3 und/oder αvβ5, insbesondere von αvβ3, induziert eine Apoptose der aktivierten Endothelzellen des Blutgefäßes, das in den Tumor hineinwächst, während das ruhende normale Gefäßbett intakt bleibt. Der Tumor verliert seine Nahrungszufuhr aufgrund dessen seine Entwicklung gestoppt wird. Der Tumor an sich, d.h. die entarteten Krebszellen, werden bei diesem Therapieansatz jedoch nicht beeinträchtigt, so daß das Wachstum des Tumors nach Abbruch der Behandlung wieder einsetzen kann.

Die Integrinrezeptoren auf der Oberfläche von Tumorzellen unterscheiden sich signifikant von denjenigen die auf normalem Gewebe exprimiert werden. Beispielsweise wird auf vielen Karzinomen eine hohe de-novo-Expression des seltenen Integrins αvβ6 gefunden, während αvβ3 ein guter Marker für fortschreitende maligne Melanome ist. Weiterhin ist bekannt, daß αvβ6 in die Mechanismen der eigentlichen Tumorentwicklung und in die Mechanismen des Befalls von gesundem Gewebe durch entartete Zellen, der Metastasierung, eingreift.

Eine kombinierte Therapie, die gleichzeitig die Angiogenese des Tumorgewebes und die Bekämpfung des Tumorgewebes an sich als Angriffspunkt hat wurde bisher nur über den kombinierten Einsatz von αvβ3-Integrininhibitoren mit cytotoxischen Substanzen (Chemotherapie) oder durch Bestrahlung (Radiotherapie) beschrieben.

Überraschenderweise wurde gefunden, daß der Einsatz von selektiven αvβ3-Integrininhibitoren und der Einsatz von selektiven αvβ6-Integrininhibitoren zur Herstellung eines Arzneimittels für eine solche kombinierte Therapie geeignet ist. Es wird ein synergistischer Effekt beobachtet. Durch die hohe Selektivität der Inhibierung des αvβ3- und αvβ6-Integrinrezeptors bleibt eine weitere Inhibierung von anderen Integrinen - wie beispielsweise α5β1 oder αIIbβ3 - aus, die z.B. in normalem Gewebe wichtige und kritische Funktionen haben.

Es wurde weiterhin gefunden, daß der Einsatz von selektiven αvβ5-Integrininhibitoren und der Einsatz von selektiven αvβ6-Integrininhibitoren zur Herstellung eines Arzneimittels für eine solche kombinierten Therapie ebenfalls geeignet ist sowie der kombinierte Einsatz von selektiven αvβ3-, αvβ5 und αvβ6-Integrininhibitoren. Es wird jeweils ein synergistischer Effekt beobachtet.

Die Verbindungen der Formel I besitzen mindestens ein chirales Zentrum und können daher in mehreren stereoisomeren Formen auftreten. Alle diese Formen (z.B. D- und L-Formen) und deren Gemische (z.B. die DL-Formen) sind in der Formel eingeschlossen.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Gegenstand der Erfindung sind die Verbindungen der Formel I und ihre Salze und Solvate nach Anspruch 1 sowie ein Verfahren zur Herstellung von Verbindungen der Formel I sowie ihrer Salze und Solvate, dadurch gekennzeichnet, daß man
(a) eine Verbindung der Formel II 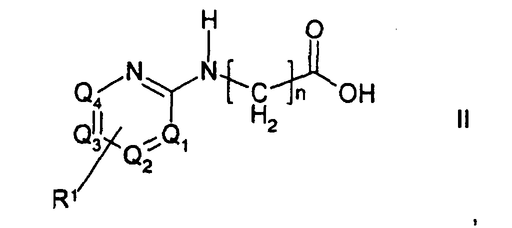 worin Q₁, Q₂, Q₃, Q₄, R¹ und n die in Anspruch 1 angegebenen Bedeutungen haben,
   mit einer Verbindung der Formel III 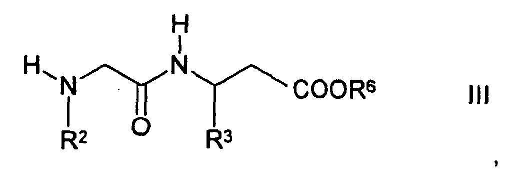 worin R², R³, R⁴, R⁵ und R⁶ die in Anspruch 1 angegebenen Bedeutungen haben
   umsetzt
   und gegebenenfalls den Rest R⁶ ≠ H in den Rest R⁶ = H
   umwandelt,
   oder
(b) eine Verbindung der Formel IV 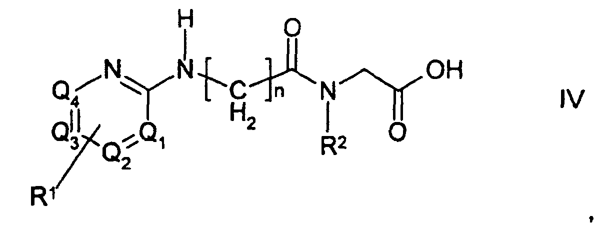 worin Q₁, Q₂, Q₃, Q₄, R¹, R² und n die in Anspruch 1 angegebenen Bedeutungen haben,
   mit einer Verbindung der Formel V 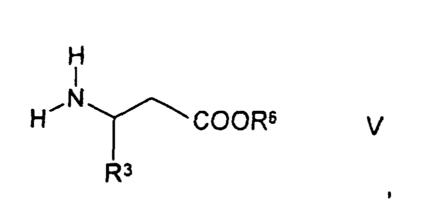 worin R³, R⁴, R⁵ und R⁶ die in Anspruch 1 angegebenen Bedeutungen haben
   umsetzt
   und gegebenenfalls den Rest R⁶ ≠ H in den Rest R⁶ = H
   umwandelt,
   oder
(c) in einer Verbindung der Formel I einen oder mehrere Reste R¹, R², R³, R⁴ und/oder R⁵ in einen oder mehrere Reste R¹, R², R³, R⁴ und/oder R⁵ umwandelt,
   indem man beispielsweise
   i) eine Hydroxygruppe alkyliert,
   ii) eine Estergruppe zu einer Carboxygruppe hydrolysiert,
   iii) eine Carboxygruppe verestert,
   iv) eine Aminogruppe alkyliert oder
   v) eine Aminogruppe acyliert,
und/oder
eine basische oder saure Verbindung der Formel I durch Behandeln mit einer Säure oder Base in eines ihrer Salze oder Solvate umwandelt.

In den vorstehenden Formeln bedeutet A Alkyl, ist linear oder verzweigt, und hat 1 bis 6, vorzugsweise 1, 2, 3, 4, 5 oder 6 C-Atome. A bedeutet vorzugsweise Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Besonders bevorzugt für A ist Methyl, Ethyl, Isopropyl, n-Propyl, n-Butyl oder tert.-Butyl.

Ar bedeutet unsubstituiertes oder ein-, zwei- oder dreifach durch A, CF₃, OH, OA, OCF₃, CN, NO₂ oder Hal substituiertes Aryl, wobei Aryl Phenyl, Naphthyl, Anthryl oder Biphenylyl bedeutet. Vorzugsweise ist Ar unsubstituiertes oder ein-, zwei- oder dreifach druch A, CF₃, OH, OA, OCF₃, CN, NO₂ oder Hal substituiertes Phenyl oder Naphthyl.

Ar bedeutet daher bevorzugt Phenyl, o-, m- oder p-Methylphenyl, o-, m-oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Ethoxyphenyl, o-, m-, p-Trifluormethylphenyl, o-, m- oder p-Fluorphenyl, o-, m- oder p-Chlorphenyl, o-, m- oder p-Bromphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl oder 3-Chlor-4-Fluorphenyl, 4-Fluor-2-hydroxyphenyl, Naphthalin-1-yl, Naphthalin-2-yl oder 2-, 3-, 4-, 5-, 6-, 7- oder 8-Methyl-naphthalin-1-yl, 2-, 3-, 4-, 5-, 6-, 7- oder 8-Ethyl-naphthalin-1-yl, 2-, 3-, 4-, 5-, 6-, 7- oder 8-Chlor-naphthalin-1-yl, 2-, 3-, 4-, 5-, 6-, 7- oder 8-Fluor-naphthalin-1-yl, 2-, 3-, 4-, 5-, 6-, 7- oder 8-Brom-naphthalin-1-yl, 2-, 3-, 4-, 5-, 6-, 7- oder 8-Hydroxy-naphthalin-1-yl, 1-, 3-, 4-, 5-, 6-, 7- oder 8-Methyl-naphthalin-2-yl, 1-, 3-, 4-, 5-, 6-, 7- oder 8-Ethyl-naphthalin-2-yl, 1-, 3-, 4-, 5-, 6-, 7- oder 8-Chlor-naphthalin-2-yl, 1-, 3-, 4-, 5-, 6-, 7- oder 8-Fluor-naphthalin-2-yl, 1-, 3-, 4-, 5-, 6-, 7- oder 8-Brom-naphthatin-2-yl, 1-, 3-, 4-, 5-, 6-, 7- oder 8-Hydroxy-naphthalin-2-yl.
Ganz besonders bevorzugt ist Ar Phenyl, o-, m- oder p-Fluorphenyl, m-oder p-Chlorphenyl, p-Methylphenyl, p-Trifluormethylphenyl, 3-Chlor-4-fluorphenyl, 4-fluor-2-hydroxyphenyl, Naphthalin-1-yl oder Naphthalin-2-yl.

In -(CH₂)ₘ-Ar hat Ar eine der zuvor angegebenen bevorzugten Bedeutungen, wobei m 1 oder 2 sein kann. Besonders bevorzugt für -(CH₂)ₘ-Ar ist Benzyl.

In -(CH₂)ₘ-OH kann m 1, 2, 3 oder 4 sein. Vorzugsweise ist -(CH₂)ₘ-OH Hydroxymethyl, Hydroxyethyl, Hydroxypropyl oder Hydroxybutyl, ganz besonders bevorzugt Hydroxyethyl.

In -(CH₂)ₘ-O-C(O)A hat A eine der zuvor angegebenen bevorzugten Bedeutungen, wobei m 1 oder 2 sein kann. Besonders bevorzugt ist A tert.-Butyl und m 1.

Hal bedeutet vorzugsweise F, Cl oder Brom.

n bedeutet 2, 3, 4, 5 oder 6, besonders bevorzugt 3, 4 oder 5. 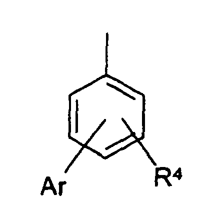 bedeutet bevorzugt Biphenyl-4-yl, 4'-Fluorbiphenyl-4-yl, 4'-Fluorbiphenyl-3-yl, 3'-Fluorbiphenyl-4-yl, 2'-Fluorbiphenyl-4-yl, 4'-Chlorbiphenyl-4-yl, 3'-Chlorbiphenyl-4-yl, 4'-Methylbiphenyl-4-yl, 4'-(Trifluormethyl)biphenyl-4-yl, 3'-Chlor-4'-fluor-biphenyl-4-yl, 4'-Fluor-2-hydroxy-biphenyl-4-yl, 4'-Fluor-2'-hydroxy-biphenyl-4-yl, 4'-Fluor-2'-hydroxy-biphenyl-3-yl, 4-(Naphthalin-1-yl)-phenyl, 4-(Naphthalin-2-yl)-phenyl oder 4-(Naphthalin-1-yl)-3-hydroxyphenyl.

Q₁, Q₂, Q₃ oder Q₄ sind jeweits unabhängig voneinander CH oder N. Q₁ ist bevorzugt CH oder N, Q₂ ist bevorzugt CH, Q₃ ist bevorzugt CH und Q₄ ist bevorzugt CH. Ganz besonders bevorzugt sind Q₁, Q₂, Q₃ und Q₄ CH.

R¹ bedeutet H, A, Ar, Hal, OH, OA, CF₃ oder OCF₃, wobei A, Ar oder Hal die zuvor angegebenen bevorzugten oder besonders bevorzugten Bedeutungen haben. R¹ bedeutet bevorzugt H oder A. Die bevorzugten Stellungen des Substituenten R¹ sind die 4- oder 6-Position. Besonders bevorzugt die 4-Position des Ringsystems.

R² ist vorzugsweise H oder A, besonders bevorzugt H.

R³ bedeutet. 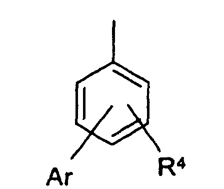 wobei R⁴ H, A, Hal, OH, OA, CF₃, OCF₃, CN, NH₂, NHA, NA₂ oder NH-C(O)A bedeutet und Ar eine der zuvor angegebenen Bedeutungen hat. Besonders bevorzugt für R⁴ ist H, A, OH oder Hal.

Besonders bevorzugt is R⁴ H oder OH und Ar unsubstituiertes oder durch Hal, A oder CF₃ ein- oder zweifach substituiertes Phenyl oder unsubstituiertes Naphthyl in

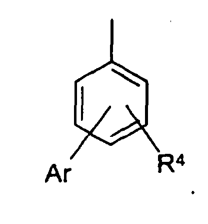

R³ ist vorzugsweise Biphenyl-3-yl, Biphenyl-4-yl, 4'-Fluorbiphenyl-4-yl, 4'-Fluorbiphenyl-3-yl, 3'-Fluorbiphenyl-4-yl, 2'-Fluorbiphenyl-4-yl, 4'-Chlorbiphenyl-4-yl, 3'-Chlorbiphenyl-4-yl, 4'-Methylbiphenyl-4-yl, 4'-(Trifluormethyl)biphenyl-4-yl, 3'-Chlor-4'-fluor-biphenyl-4-yl, 4'-Fluor-2-hydroxy-biphenyl-4-yl, 4'-Fluor-2'-hydroxy-biphenyl-4-yl, 4'-Fluor-2'-hydroxybiphenyl-3-yl, 4-(Naphthalin-1-yl)-phenyl, 4-(Naphthalin-2-yl)-phenyl oder 4-(Naphthalin-1-yl)-3-hydroxyphenyl.

Besonders bevorzugte Bedeutungen für R³ sind Biphenyl-4-yl, 4'-Fluorbiphenyl-4-yl, 4'-Fluorbiphenyl-3-yl, 3'-Fluorbiphenyl-4-yl, 2'-Fluorbiphenyl-4-yl, 4'-Chlorbiphenyl-4-yl, 3'-Chlorbiphenyl-4-yl, 4'-Methylbiphenyl-4-yl, 4'-(Trifluormethyl)biphenyl-4-yl, 3'-Chlor-4'-fluorbiphenyl-4-yl, 4'-Fluor-2-hydroxy-biphenyl-4-yl, 4'-Fluor-2'-hydroxy-biphenyl-4-yl, 4'-Fluor-2'-hydroxy-biphenyl-3-yl, 4-(Naphthalin-1-yl)-phenyl, 4-(Naphthalin-2-yl)-phenyl oder 4-(Naphthalin-1-yl)-3-hydroxyphenyl.

R⁶ bedeutet H, A, -(CH₂)ₘ-OH, -(CH₂)ₘ-O-C(O)A oder -(CH₂)ₘ-Ar, wobei A, -(CH₂)ₘ-OH, -(CH₂)ₘ-O-C(O)A und -(CH₂)ₘ-Ar eine der zuvor angegebenen bevorzugten oder besonders bevorzugten Bedeutungen haben.

Verbindungen der Formel I, bei denen R⁶ vorzugsweise A, -(CH₂)ₘ-OH, -(CH₂)ₘ-O-C(O)A oder -(CH₂)ₘ-Ar ist sowie deren Solvate, sind sogenannte Prodrugs, d.h. sie sind in in-vitro-Versuchen inaktiv, da sie die biologisch aktive Carboxylgruppe maskieren. Prodrugs werden jedoch im Körper in die biologisch aktive Form metabolisch umgewandelt. Die entsprechende freie Säure, die einer Verbindung der Formel I mit R⁶ = H entspricht sowie ihre Salze und Solvate ist in-vitro aktiv.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ik ausgedrückt werden, die der Formel I entsprechen und worin die nicht näher bezeichneten Reste die bei der Formel I angegebene Bedeutung haben, worin jedoch

In la
R3 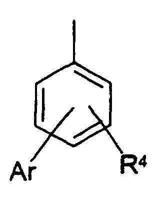 bedeutet.

In Ib Q₁, Q₂, Q₃ und Q₄ der Formel I CH bedeuten.

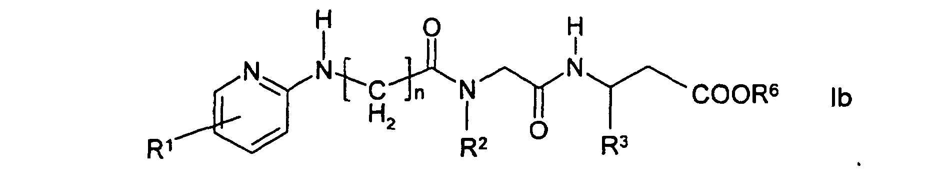

In Ic Q₁ N bedeutet und Q₂, Q₃ und Q₄ der Formel I CH bedeuten.

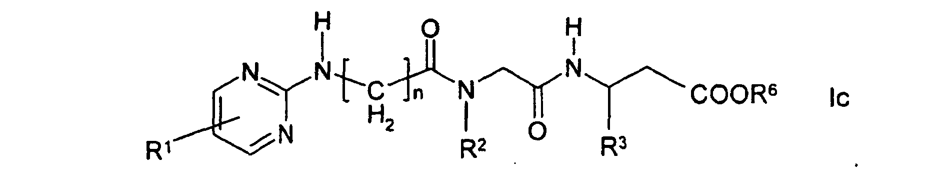

In Id R¹ H oder A und
R3 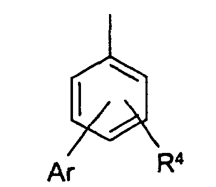 bedeutet.

In le R¹ H oder A,
R3 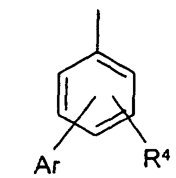 und
Q₁ N bedeutet und Q₂, Q₃ und Q₄ CH bedeuten.

In If R¹ H oder A,
Q₁, Q₂, Q₃ und Q₄ CH und
R3 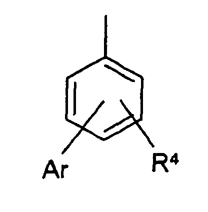 bedeutet.

Besonders bevorzugte Verbindungen der Formel la sind
3-{2-[4-(4-Methyl-pyridin-2-ylamino)-butyrylamino]-acetylamino}-3-(4-biphenylyl)-propionsäure,
3-{2-[4-(Pyridin-2-ylamino)-butyrylamino]-acetylamino}-3-(4-biphenylyl)-propionsäure,
3-{2-[5-(Pyrimidin-2-ylamino)-pentanoylamino]-acetylamino}-3-(4-biphenylyl)-propionsäure
3-{2-[4-(Pyrimidin-2-ylamino)-butyrylamino]-acetylamino}-3-(4-biphenylyl)-propionsäure,
3-{2-[5-(Pyridin-2-ylamino)-pentanoylamino]-acetylamino}-3-(4-biphenylyl)-propionsäure oder
sowie ihre physiologisch unbedenklichen Salze und Solvate.

Besonders bevorzugte Verbindungen der Formel Ib sind
3-{2-[4-(4-Methyl-pyridin-2-ylamino)-butyrylamino]-acetylamino}-3-(4-biphenylyl)-propionsäure,
3-{2-[4-(Pyridin-2-ylamino)-butyrylamino]-acetylamino}-3-(4-biphenylyl)-propionsäure,
3-{2-[5-(Pyridin-2-ylamino)-pentanoylamino]-acetylamino}-3-(4-biphenylyl)-propionsäure oder
3-{2-[5-(4-Methyl-pyridin-2-ylamino)-pentanoylamino]-acetylamino}-3-(4-biphenylyl)-propionsäure,
sowie ihre physiologisch unbedenklichen Salze und Solvate.

Bevorzugte Verbindungen der Formel Ic sind
3-{2-[5-(Pyrimidin-2-ylamino)-pentanoylamino]-acetylamino}-3-(4-biphenylyl)-propionsäure oder
3-{2-[4-(Pyrimidin-2-ylamino)-butyrylamino]-acetylamino}-3-(4-biphenylyl)-propionsäure,
sowie ihre physiologisch unbedenklichen Salze und Solvate.

Bevorzugte Verbindungen der Formel Id sind
3-{2-[4-(Pyridin-2-ylamino)-butyrylamino]-acetylamino}-3-(4-biphenylyl)-propionsäure,
3-{2-[5-(4-Methyl-pyridin-2-ylamino)-pentanoylamino]-acetylamino}-3-(4-biphenylyl)-propionsäure,
3-{2-[5-(Pyridin-2-ylamino)-pentanoylamino]-acetylamino}-3-(4-biphenylyl)-propionsäure oder
3-{2-[4-(4-Methyl-pyridin-2-ylamino)-butyrylamino]-acetylamino}-3-(4-biphenylyl)-propionsäure,
sowie ihre physiologisch unbedenklichen Salze und Solvate.

Die Verbindungen der Formel I nach Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I nach Anspruch 1 umsetzt.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden (vgl. dazu: T.W. Greene, P.G.M. Wuts, *Protective Groups in Organic Chemistry,* 2. Aufl., Wiley, New York **1991** oder P.J. Kocienski, *Protecting Groups*, 1. Aufl., Georg Thieme Verlag, Stuttgart - New-York, **1994**, H. Kunz, H. Waldmann in *Comprehensive Organic Synthesis,* Vol. 6 (Hrsg. B.M. Trost, I. Fleming, E. Winterfeldt), Pergamon, Oxford, **1991**, S. 631-701).

Der Ausdruck "Aminoschutzgruppe" ist allgemein bekannt und bezieht sich auf Gruppen, die geeignet sind, eine Aminogruppe vor chemischen Umsetzungen zu schützen (zu blockieren). Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, alicyclischen, aromatischen oder heterocyclischen Carbonsäuren oder Sulfonsäuren abgeleitete Acylgruppen sowie insbesondere Alkoxycarbonyl-, Alkenyloxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichiorethoxy-carbonyl, BOC, 2-lodethoxycarbonyl; Alkenyloxycarbonyl wie Allyloxycarbonyl (Aloc), Aralkyloxycarbonyl wie CBZ (synonym mit Z), 4-Methoxybenzyloxycarbonyl (MOZ), 4-Nitro-benryloxycarbonyl oder 9-fluorenylmethoxycarbonyl (Fmoc); 2-(Phenylsulfonyl)ethoxycarbonyl; Trimethylsilylethoxycarbonyl (Teoc) oder Arylsulfonyl wie 4-Methoxy-2,3,6-trimethylphenyl-sulfonyl (Mtr). Bevorzugte Aminoschutzgruppen sind BOC, Fmoc und Aloc, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen. Typisch für solche Gruppen sind die oben genannten unsubstituierten oder substituierten Aryl-, Aralkyl-, Aroyl- oder Acylgruppen, ferner auch Alkylgruppen, Alkyl-, Aryl- oder Aralkyl-silylgruppen oder O,O- oder O,S-Acetale. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Aralkylgruppen wie Benzyl, 4-Methoxybenzyl oder 2,4-Dimethoxybenzyl, Aroylgruppen wie Benzoyl oder p-Nitrobenzoyl, Acylgruppen wie Acetyl oder Pivaloyl, p-Toluolsulfonyl, Alkylgruppen wie Methyl oder tert.-Butyl, aber auch Allyl, Alkylsilylgruppen wie Trimethylsilyl (TMS), Triisopropylsilyl (TIPS), tert.-Butyldimethylsilyl (TBS) oder Triethylsilyl, Trimethylsilylethyl, Aralkylsilylgruppen wie tert.-Butyldiphenylsilyl (TBDPS), cyclische Acetale wie Isopropyliden-, Cyclopentyliden-, Cyclohexyliden-, Benzyliden-, p-Methoxybenzyliden- oder o,p-Dimethoxybenzylidenacetal, acyclische Acetale wie Tetrahydropyranyl (Thp), Methoxymethyl (MOM), Methoxyethoxymethyl (MEM), Benzyloxymethyl (BOM) oder Methylthiomethyl (MTM). Besonders bevorzugte Hydroxyschutzgruppen sind Benzyl, Acetyl, tert.-Butyl oder TBS.

Das In-Freiheit-Setzen der Verbindungen der Formel I aus ihren funktionellen Derivaten ist für die jeweils benutzte Schutzgruppe aus der Literatur bekannt (z.B. T.W. Greene, P.G.M. Wuts, *Protective Groups in Organic Chemistry,* 2. Aufl., Wiley, New York **1991** oder P.J. Kocienski, *Protecting Groups,* 1. Aufl., Georg Thieme Verlag, Stuttgart - New-York, **1994**). Dabei kann man auch von an sich bekannten, hier nicht näher erwähnten Varianten Gebrauch machen.

Die Gruppen BOC und O-tert.-Butyt können z.B. bevorzugt mit TFA in Dichlormethan oder mit etwa 3 bis 5N HCl in Dioxan bei 15-30°C abgespalten werden, die Fmoc-Gruppe mit einer etwa 5- bis 50%igen Lösung von Dimethylamin, Diethylamin oder Piperidin in DMF bei 15-30°C. Die Aloc-Gruppe läßt sich schonend unter Edelmetallkatalyse in Chloroform bei 20-30°C spalten. Ein bevorzugter Katalysator ist Tetrakis(triphenyl-phosphin)palladium(0).

Die Ausgangsverbindungen der Formel II bis V sind in der Regel bekannt. Sind sie neu, so können sie aber nach an sich bekannten Methoden hergestellt werden.

Verbidungen der Formel II werden beispielsweise aus einer Kupplung der entsprechenden 2-Amino-Verbindung des Heterocyclus, worin Q₁, Q₂, Q₃ oder Q₄ die in Anspruch 1 angegebenen Bedeutungen haben, mit den entsprechenden n-Bromcarbonsäureestern (Br-[CH₂]ₙ-COOSG¹, wobei SG¹ eine Hydroxyschutzgruppe bedeutet wie zuvor beschrieben) in Gegenwart einer Base und anschließender Abspaltung der Schutzgruppe unter Standardbedingungen erhalten.

Verbindungen der Formel IV werden durch eine peptidanaloge Kupplung der Verbindungen der Formel II mit einem Glycinderivat H₂N-CH₂-COOSG², wobei SG² eine Hydroxyschutzgruppe bedeutet wie zuvor beschrieben, unter Standardbedingungen erhalten.

Verbindungen der Formel V (β-Aminosäuren) können analog zu Skinner et al., J. Org. Chem. **1960**, 25, 1756 hergestellt werden. Die Umsetzung des entsprechenden Aldehyds R³-CHO mit Malonsäure und Ammoniumacetat in einem geeigneten Lösungsmittel, besonders bevorzugt sind Alkohole wie z.B. Ethanol, erzeugt die β-Aminosäure der Formel V, wobei R⁶ H bedeutet. Eine Veresterung dieser freien Säure der Formel V unter Standardbedingungen ergibt Verbindungen der Formel V, wobei R⁶ A oder -(CH₂)ₘ-Ar bedeutet.

Zur Herstellung der Verbindungen der Formel III werden die an der Säurefunktion geschützten β-Aminosäuren der Formel V, entweder durch eine entsprechende Schutzgruppe oder wenn R⁶ A, -(CH₂)ₘ-OH, -(CH₂)ₘ-O-C(O)A oder -(CH₂)ₘ-Ar bedeutet, mit einem Glycinderivat SG³-NH-CH₂-COOH gekuppelt. Der Substituent SG³ des Glycinderivats SG³-NH-CH₂-COOH bedeutet eine Aminoschutzgruppe, wie zuvor beschrieben, die anschließend abgespalten wird. Übliche Methoden der Peptidsynthese werden z.B. in Houben-Weyl, 1.c., Band 15/II, **1974**, Seite 1 bis 806 beschrieben.

Verbindungen der Formel I können erhalten werden, indem man eine Verbindung der Formel II mit einer Verbindung der Formel III umsetzt und anschließend eine Schutzgruppe abspaltet oder den Rest R⁶, der A, -(CH₂)ₘ-OH, -(CH₂)ₘ-O-C(O)A oder -(CH₂)ₘ-Ar bedeutet, in den Rest R⁶ = H umwandelt.

Die Verbindungen der Formel I können ebenfalls erhalten werden, indem man eine Verbindung der Formel IV mit einer Verbindung der Formel V umsetzt und anschließend eine Schutzgruppe abspaltet oder den Rest R⁶, der A, -(CH₂)ₘ-OH, -(CH₂)ₘ-O-C(O)A oder -(CH₂)ₘ-Ar bedeutet, in den Rest R⁶ = H umwandelt.

Die Kupplungsreaktion gelingt vorzugsweise in Gegenwart eines Dehydratisierungsmittels, z.B. eines Carbodiimids wie Dicyclohexylcarbodiimid (DCC), N-(3-Dimethylaminopropyl)-N'-ethylcarbodiimid-hydrochlorid (EDC) oder Diisopropylcarbodiimid (DIC), ferner z.B. Propanphosphonsäureanhydrid (vgl. Angew. Chem. **1980**, *92*, 129), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30°. Die Reaktionszeit liegt je nach den angewendeten Bedingungen zwischen einigen Minuten und mehreren Tagen.
Als besonders vorteilhaft hat sich die Zugabe des Kupplungsreagenzes TBTU (O-(Benzotriazol-1-yl)-N,N,N',N'-tetramethyl-uroniumtetrafluoroborat) oder O-(Benzotriazol-1-yl)-N,N,N',N'-tetramethyl-uroniumhexafluorophosphat erwiesen, da in Gegenwart einer dieser Verbindungen nur eine geringe Racemisierung auftritt und keine cytotoxischen Nebenprodukte entstehen.

Anstelle von Verbindungen der Formeln II und/oder IV können auch Derivate von Verbindungen der Formel II und/oder IV, vorzugsweise eine voraktivierte Carbonsäure, oder ein Carbonsäurehalogenid, ein symmetrisches oder gemischtes Anhydrid oder ein Aktivester eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben. Aktivierte Ester werden zweckmäßig in situ gebildet, z.B. durch Zusatz von HOBt (1-Hydroxybenzotriazol) oder N-Hydroxysuccinimid.

Die Umsetzung erfolgt in der Regel in einem inerten Lösungsmittel, bei Verwendung eines Carbonsäurehalogenids in Gegenwart eines säurebindenden Mittels vorzugsweise einer organischen Base wie Triethylamin, Dimethylanilin, Pyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden. Andererseits können Verbindungen der Formel I mit Basen (z.B. Natriumoder Kaliumhydroxid oder -carbonat) in die entsprechenden Metall-, insbesondere Alkalimetall- oder Erdalkalimetall- oder in die entsprechenden Ammoniumsalze umgewandelt werden.

Gegenstand der Erfindung sind auch die Verbindungen der Formel I nach Anspruch 1 und ihre physiologisch unbedenklichen Salze oder Solvate als Arzneimittelwirkstoffe.

Weiterhin sind Gegenstand der Erfindung Verbindungen der Formel I nach Anspruch 1 und ihre physiologisch unbedenklichen Salze oder Solvate als Integrininhibitoren.

Gegenstand der Erfindung sind auch die Verbindungen der Formel I nach Anspruch 1 und ihre physiologisch unbedenklichen Salze oder Solvate zur Anwendung bei der Bekämpfung von Krankheiten.

Gegenstand der Erfindung ist ferner die Verwendung einer Kombination selektiver Integrininhibitoren, ausgewählt aus der Gruppe selektiver αvβ3-Integrininhibitoren kombiniert mit selektiven αvβ6-Integrininhibitoren, selektiver αvβ5-Integrininhibitoren kombiniert mit selektiven αvβ6-Integrininhibitoren oder selektiver αvβ3-Integrininhibitoren kombiniert mit selektiven αvβ5-Integrininhibitoren und kombiniert mit selektiven αvβ6-Integrininhibitoren, zur Herstellung eines Arzneimittels zur Bekämpfung pathologischer Vorgänge, die durch die Integrine αvβ3 und/oder αvβ5 und αvβ6 beeinflußt werden.

Gegenstand der Erfindung ist die Verwendung einer Kombination selektiver Integrininhibitoren, ausgewählt aus der Gruppe selektiver αvβ3-Integrininhibitoren kombiniert mit selektiven αvβ6-Integrininhibitoren, selektiver αvβ5-Integrininhibitoren kombiniert mit selektiven αvβ6-Integrininhibitoren oder selektiver αvβ3-Integrininhibitoren kombiniert mit selektiven αvβ5-Integrininhibitoren und kombiniert mit selektiven αvβ6-Integrininhibitoren, zur Herstellung eines Arzneimittels zur Krebstherapie, wobei zum einen durch Hemmung des αvβ3-Integrinrezeptors und/oder des αvβ5-Integrinrezeptors die Angiogenese der in den Tumor einwachsenden Blutgefäße und zum anderen durch Hemmung des αvβ6-Integrinrezeptors die Tumorentwicklung unterbunden wird.

Gegenstand der Erfindung ist die Verwendung einer Kombination selektiver αvβ3-Integrininhibitoren und/oder selektiver αvβ5- und selektiver αvβ6-Integrininhibitoren zur Herstellung eines Arzneimittels zur Bekämpfung von Krankheiten, die mit Krebs assoziiert sind, wie Metastasen von soliden Tumoren, Angiofibromatose, retrolentale Fibroplasie, Hemangioma oder Kaposi Sarkoma.

Im folgenden eine ausgewählte Verbindung der Formel I, die insbesondere ein selektiver αvβ3- und αvβ6-Integrinrezeptorinhibitor ist: 3-(Biphenyl-4-yl)-3-{2-[5-(pyridin-2-ylamino)pentanoylamino]-acetylamino}propionsäure Trifluoracetat.

Gegenstand der Erfindung ist die Verwendung von 3-(Biphenyl-4-yl)-3-{2-[5-(pyridin-2-ylamino)pentanoylamino]-acetylamino}-propionsäure Trifluoracetat zur Herstellung eines Arzneimittels zur Bekämpfung von Krankheiten, die mit Krebs assoziiert sind, wie Metastasen von soliden Tumoren, Angiofibromatose, retrolentale Fibroplasie, Hemangioma oder Kaposi Sarkoma.

Besonders geeignet für eine Verwendung zur Herstellung eines Arzneimittels zur Krebstherapie, wobei zum einen durch Hemmung des αvβ3-Integrinrezeptors die Angiogenese der in den Tumor einwachsenden Blutgefäße und zum anderen durch Hemmung des αvβ6-Integrinrezeptors die Tumorentwicklung unterbunden wird, ist das β-Alaninderivat 3-(Biphenyl-4-yl)-3-{2-[5-(pyridin-2-ylamino)pentanoylamino]-acetylamino}propionsäure Trifluoracetat.

Zusätzlich zu dem erfindungsgemäßen Einsatz der selektiven Integrinrezeptorinhibitoren als interne Kombinationstherapie von mindestens zwei Integrininhibitoren, sind auch weitere Kombinationen mit herkömmlichen Therapien, wie der Radiotherapie, der Tumorvaccinierung, Immun- oder Chemotherapie denkbar. Dieser kombinierte interne und externe Therapieansatz sollte die Effektivität der Behandlung weiter erhöhen, vor allem die Dosierung der toxischen Therapeutika senken und somit auch die dosisbezogenen Nebeneffekte reduzieren.

Gegenstand der Erfindung sind ferner pharmazeutische Zubereitungen, enthaltend mindestens eine Verbindung der Formel I und/oder eines ihrer physiologisch unbedenklichen Salze oder Solvate, die insbesondere auf nicht-chemischem Wege hergestellt werden. Hierbei können die Verbindungen der Formel I zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacksund/oder mehrere weitere Wirkstoffe enthalten, z.B. ein oder mehrere Vitamine.
Für die Applikation als Inhalationsspray können Sprays verwendet werden, die den Wirkstoff entweder gelöst oder suspendiert in einem Treibgas oder Treibgasgemisch (z.B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabreicht werden.

Die Verbindungen der Formel I und ihre physiologisch unbedenklichen Salze oder Solvate können als Integrininhibitoren bei der Bekämpfung von Krankheiten, insbesondere von Thrombosen, Herzinfarkt, koronaren Herzerkrankungen, Arteriosklerose, Tumoren, Osteoporose, Entzündungen und Infektionen verwendet werden.

Die Verbindungen der Formel I nach Anspruch 1 und/oder ihre physiologisch unbedenklichen Salze finden auch Verwendung bei pathologischen Vorgängen, die durch Angiogenese unterhalten oder propagiert werden, insbesondere bei Tumoren oder rheumatoider Arthritis.

Ausgewählte Verbindungen der Formel I und/oder ihre Solvate, wie zuvor beschrieben, finden Verwendung bei der Bekämpfung pathologischer Vorgänge, die durch die Integrine αvβ3 und/oder αvβ5 und αvβ6 beeinflußt werden, insbesondere der Krebstherapie, wobei zum einen durch Hemmung des αvβ3-Integrinrezeptors und/oder des αvβ5-Integrinrezeptors die Angiogenese der in den Tumor einwachsenden Blutgefäße und zum anderen durch Hemmung des αvβ6-Integrinrezeptors die Tumorentwicklung unterbunden wird.

Dabei werden die erfindungsgemäßen Substanzen in der Regel in Analogie zu den in WO 97/26250 oder WO 97/24124 beschriebenen Verbindungen verabreicht, vorzugsweise in Dosierungen zwischen etwa 0,05 und 500 mg, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die Verbindungen der Formel I als Integrinliganden zur Herstellung von Säulen für die Affinitätschromatographie zur Reindarstellung von Integrinen verwendet werden.
Der Ligand, d.h. eine Verbindung der Formel I, wird dabei über eine Ankerfunktion, z.B. die Carboxygruppe, an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervernetzte Polyzucker wie Cellulose, Sepharose oder Sephadex^{R}, Acrylamide, Polymer auf Polyethylenglykolbasis oder Tentakelpolymere^{R}.

Die Herstellung der Materialien für die Affinitätschromatographie zur Integrinreinigung erfolgt unter Bedingungen, wie sie für die Kondensation von Aminosäuren üblich und an sich bekannt sind.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt duch Chromatographie an Kieselgel, durch präparative HPLC und/oder durch Kristallisation. Die gereinigten Verbindungen werden gegebenenfalls gefriergetrocknet.

RT = Retentionszeit (in Minuten) bei HPLC in den folgenden Systemen:
Säule: Lichrosorb RP-18 (5 µm) 250 x 4 mm;
Lichrosorb RP-18 (15 µm) 250 x 50 mm.
Als Eluenten kommen Gradienten aus Acetonitril (B) mit 0,1 % TFA (Trifluoressigsäure) und Wasser (A) mit 0,1 % TFA zum Einsatz. Der Gradient wird in Volumenprozent Acetonitril angegeben.
Bevorzugter Gradient: 5 min bei 20 % B und 55 min auf 90 % B.
Detektion bei 225 nm.
Die mit * gekennzeichneten Retentionszeiten wurden mit dem Gradienten 5 min bei 5% B und 40 min bis zu 80% B gemessen.
Die durch präparative HPLC gereinigten Verbindungen werden als Trifluoracetate isoliert.

Massenspektrometrie (MS) mittels FAB (Fast Atom Bombardment): MS-FAB (M+H)⁺.

### Beispiel 2:

(1) 0,06 mol 2-Nitro-benzaldehyd, 5,72 g Malonsäure, 8,5 g Ammoniumacetat und 40 ml Ethanol werden 8 Stunden unter Rückfluß gekocht und über Nacht bei Raumtemperatur gerührt. Danach wird das abgekühlte Reaktionsgemisch abgesaugt, mit Ethanol und Ether gewaschen und an der Luft getrocknet. Man erhält 3-Amino-3-(2-nitrophenyl)-propionsäure, Smp. 222°.
   Die anschließende Veresterung durch Aktivierung mit Thionylchlorid und Reaktion mit Methanol unter Standardbedingungen erzeugt 3-Amino-3-(2-nitro-phenyl)-propionsäuremethylester.
(2) 3-Amino-3-(2-nitro-phenyl)-propionsäuremethylester wird analog zu Beispiel 1 (2) mit BOC-geschütztem Glycin (BOC-Gly-OH) umgesetzt. Nach Abspaltung der Aminoschutzgruppe BOC unter Standardbedingungen erhält man 3-(2-Amino-acetylamino)-3-(2-nitrophenyl)-propionsäuremethyiester Hydrochlorid, FAB-MS (M+H)⁺ 317.
(3) 360 mg 3-(2-Amino-acetylamino)-3-(2-nitro-phenyl)-propionsäuremethylester Hydrochlorid werden in 35 ml DMF gelöst und 190 mg 4-(4-Methyl-pyridin-2-ylamino)-buttersäure hinzugegeben. Nach Kühlung auf 0° werden dem Reaktionsgemisch 450 mg TBTU und 63 mg HOBt zugegeben. Man neutralisiert mit 0,15 ml N-Methylmorpholin und rührt über Nacht bei Raumtemperatur. Das Lösungsmittel wird abdestilliert und der Rückstand mit 30 ml Ethylacetat versetzt und mit halbkonzentrierter Bicarbonatlösung und gesättigter Kochsalzlösung gewaschen und weiter wie üblich aufgearbeitet. Man erhält 3-{2-[4-(4-Methyl-pyridin-2-ylamino)-butyrylamino]-acetylamino}-3-(2-nitro-phenyl)-propionsäuremethylester.
(4) 280 mg 3-{2-[4-(4-Methyl-pyridin-2-ylamino)-butyrylamino]-acetylamino}-3-(2-nitro-phenyl)-propionsäuremethylester (Rohmaterial) werden in 30 ml Dioxan gelöst und 0,61 ml NaOH (2 mol/l) zugegeben. Nach Rühren über Nacht bei Raumtemperatur wird das Lösungsmittel abedestilliert und wie üblich aufgearbeitet. Man erhält 3-{2-[4-(4-Methylpyridin-2-ylamino)-butyrylamino]-acetylamino}-3-(2-nitro-phenyl)-propionsäure.
   Wird mit einem Überschuß an NaOH gearbeitet, so erhält man das Natriumsalz der 3-{2-[4-(4-Methyl-pyridin-2-ylamino)-butyrylamino]-acetylamino}-3-(2-nitro-phenyl)-propionsäure.

Durch präparative HPLC erhält man 3-{2-[4-(4-Methyl-pyridin-2-ylamino)-butyrylamino]-acetylamino}-3-{2-nitro-phenyl)-propionsäure Trifluoracetat,
RT 16.14 min, FAB-MS (M+H)⁺ 444.

### Beispiel 3:

Analog zu Beispiel 2 erhält man aus 4-(4-Methyl-pyridin-2-ylamino)-buttersäure "AB"
mit 3-(2-Amino-acetylamino)-3-(4-biphenylyl)-propionsäuremethylester Hydrochlorid
   3-{2-[4-(4-Methyl-pyridin-2-ylamino)-butyrylamino]-acetylamino}-3-(4-biphenylyl)-propionsäuremethylester.

### Beispiel 4:

Nach Verseifung des Methylesters der Verbindungen von Beispiel 3 analog zu Beispiel 2 (4) erhält man
3-{2-[4-(4-Methyl-pyridin-2-ylamino)-butyrylamino]-acetylamino}-3-(4-biphenylyl)-propionsäure,
3-{2-[4-(4-Methyl-pyridin-2-ylamino)-butyrylamino]-acetylamino}-3-(4-biphenylyl)-propionsäure Natriumsalz,
3-{2-[4-(4-Methyl-pyridin-2-ylamino)-butyrylamino]-acetylamino}-3-(4-biphenylyl)-propionsäure Trifluoracetat, RT 25.65 min, FAB-MS (M+H)⁺ 475.

### Beispiel 5:

Analog zu Beispiel 2 erhält man aus 5-(4-Methyl-pyridin-2-ylamino)-pentansäure "BC"
mit 3-(2-Amino-acetylamino)-3-(4-naphthalen-2-yl-phenyl)-propionsäuremethylester Hydrochlorid
   3-(4-Naphthalen-2-yl-phenyl)-3-{2-[5-(4-methyl-pyridin-2-ylamino)-pentanoylamino]-acetylamino}-propionsäuremethylester;
mit 3-(2-Amino-acetylamino)-3-(4-biphenylyl)-propionsäuremethylester Hydrochlorid
   3-Biphenyl-4-yl-3-{2-[5-(4-Methyl-pyridin-2-ylamino)-pentanoylamino]-acetylamino}-propionsäuremethylester; nach präparativer HPLC erhält man 3-Biphenyl-4-yl-3-{2-[5-(4-Methyl-pyridin-2-ylamino)-pentanoylamino]-acetylamino}-propionsäuremethylester Trifluoracetat, RT 30.13 min, FAB-MS (M+H)⁺ 503.

### Beispiel 6:

Nach Verseifung der Methylester der Verbindungen von Beispiel 5 analog zu Beispiel 2 (4) erhält man
3-(4-Naphthalen-2-yl-phenyl)-3-{2-[5-(4-methyl-pyridin-2-ylamino)-pentanoylamino]-acetylamino}-propionsäure,
3-{4-Naphthalen-2-yl-phenyl)-3-{2-[5-(4-methyl-pyridin-2-ylamino)-pentanoylamino]-acetylamino}-propionsäure Natriumsalz,
3-(4-Naphthaten-2-yl-phenyl)-3-{2-[5-(4-methyl-pyridin-2-ylamino)-pentanoylamino]-acetylamino}-propionsäure Trifluoracetat;

3-Biphenyl-4-yl-3-{2-[5-(4-methyl-pyridin-2-ylamino)-pentanoylamino]-acetylamino}-propionsäure,
3-Biphenyl-4-yl-3-{2-[5-(4-methyl-pyridin-2-ylamino)-pentanoylamino]-acetylamino}-propionsäure Natriumsalz,
3-Biphenyl-4-yl-3-{2-[5-(4-methyl-pyridin-2-ylamino)-pentanoylamino]-acetylamino}-propionsäure Trifluoracetat, RT 27.09 min, FAB-MS (M+H)⁺ 489.

### Beispiel 7:

Analog zu Beispiel 2 erhält man aus 4-(Pyridin-2-ylamino)-buttersäure "CD"
mit 3-(2-Amino-acetylamino)-3-(4-biphenylyl)-propionsäuremethylester Hydrochlorid
   3-{2-[4-(Pyridin-2-ylamino)-butyrylamino]-acetylamino}-3-(4-biphenylyl)-propionsäuremethylester.

### Beispiel 8:

Nach Verseifung des Methylesters der Verbindung von Beispiel 7 analog zu Beispiel 2 (4) erhält man
3-Biphenyl-4-yl-3-{2-[4-(Pyridin-2-ylamino)-butyrylamino]-acetylamino}propionsäure,
3-Biphenyl-4-yl-3-{2-[4-(Pyridin-2-ylamino)-butyrylamino]-acetylamino}propionsäure Natriumsalz,
3-Biphenyl-4-yl-3-{2-[4-(Pyridin-2-ylamino)-butyrylamino]-acetylamino}propionsäure Trifluoracetat, RT 25.20 min, FAB-MS (M+H)⁺ 461.

### Beispiel 9:

Analog zu Beispiel 2 erhält man aus 5-(Pyridin-2-ylamino)-pentansäure "DE"
mit 3-(2-Amino-acetylamino)-3-(4-biphenylyl)-propionsäurernethylester Hydrochlorid
   3-Biphenyl-4-yl-3-{2-[5-(Pyridin-2-ylamino)-pentanoylamino]-acetylamino}-propionsäuremethylester, nach präparativer HPLC erhält man 3-Biphenyl-4-yl-3-{2-[5-(Pyridin-2-ylamino)-pentanoylamino]-acetylamino}propionsäuremethylester Trifluoracetat, RT 28.88 min, FAB-MS (M+H)⁺ 489.

### Beispiel 10:

Nach Verseifung des Methylesters der Verbindung von Beispiel 9 analog zu Beispiel 2 (4) erhält man
3-Biphenyl-4-yl-3-{2-[5-(Pyridin-2-ylamino)-pentanoylamino]-acetylamino}propionsäure,
3-Biphenyl-4-yl-3-{2-[5-(Pyridin-2-ylamino)-pentanoylamino]-acetylamino}propionsäure Natriumsalz,
3-Biphenyl-4-yl-3-{2-[5-(Pyridin-2-ylamino)-pentanoylamino]-acetylamino}propionsäure Trifluoracetat, RT 25.84 min, FAB-MS (M+H)⁺ 475.

### Beispiel 13:

Analog zu Beispiel 2 erhält man aus 4-(Pyrimidin-2-ylamino)-buttersäure "GH"
mit 3-(2-Amino-acetylamino)-3-(4-biphenylyl)-propionsäuremethylester Hydrochlorid
   3-{2-[4-(Pyrimidin-2-ylamino)-butyrylamino]-acetylamino}-3-(4-biphenylyl)-propionsäuremethylester.

Analog zu Beispiel 2 erhält man aus 5-(Pyrimidin-2-ylamino)-pentansäure "HK"
mit 3-(2-Amino-acetylamino)-3-(4-biphenylyl)-propionsäuremethylester Hydrochlorid
   3-{2-[5-(Pyrimidin-2-ylamino)-pentanoylamino]-acetylamino}-3-(4-biphenylyl)-propionsäuremethylester.

### Beispiel 14:

Nach Verseifung der Methylester der Verbindungen von Beispiel 13 analog zu Beispiel 2 (4) erhält man
3-Biphenyl-4-yl-3-{2-[4-(Pyrimidin-2-ylamino)-butyrylamino]-acetylamino}propionsäure,
3-Biphenyl-4-yl-3-{2-[4-(Pyrimidin-2-ylamino)-butyrylamino]-acetylamino}propionsäure Natriumsalz,
3-Biphenyl-4-yl-3-{2-[4-(Pyrimidin-2-ylamino)-butyrylamino]-acetylamino}propionsäure Trifluoracetat, RT 24.45 min, FAB-MS (M+H)⁺ 462;

3-Biphenyl-4-yl-3-{2-[5-(Pyrimidin-2-ylamino)-pentanoylamino]-acetylamino}-propionsäure,
3-Biphenyl-4-yl-3-{2-[5-(Pyrimidin-2-ylamino)-pentanoylamino]-acetylamino}-propionsäure Natriumsalz,
3-Biphenyl-4-yl-3-{2-[5-(Pyrimidin-2-ylamino)-pentanoylamino]-acetylamino}-propionsäure Trifluoracetat.

### Beispiel 18:

Analog zu Beispiel 2 erhält man aus 5-(Pyridin-2-ylamino)-pentansäure "DE"
mit 3-(2-Amino-acetylamino)-3-(4'-fluor-biphenyl-4-yl)-propionsäuremethylester Hydrochlorid
   3-(4'-Fluor-biphenyl-4-yl)-3-{2-[5-(pyridin-2-ylamino)-pentanoylamino]-acetylamino}-propionsäuremethylester;
mit 3-(2-Amino-acetylamino)-3-(4'-fluor-biphenyl-3-yl)-propionsäuremethylester Hydrochlorid
   3-(4'-Fluor-biphenyl-3-yl)-3-{2-[5-(pyridin-2-ylamino)-pentanoylamino]-acetylamino}-propionsäuremethylester;
mit 3-(2-Amino-acetylamino)-3-(3'-fluor-biphenyl-4-yl)-propionsäuremethylester Hydrochlorid
   3-(3'-Fluor-biphenyl-4-yl)-3-{2-[5-(pyridin-2-ylamino)-pentanoylamino]-acetylamino}-propionsäuremethylester;
mit 3-(2-Amino-acetylamino)-3-(2'-fluor-biphenyl-4-yl)-propionsäuremethylester Hydrochlorid
   3-(2'-Fluor-biphenyl-4-yl)-3-{2-[5-(pyridin-2-ylamino)-pentanoylamino]-acetylamino}-propionsäuremethylester;
mit 3-(2-Amino-acetylamino)-3-(4'-chlor-biphenyl-4-yl)-propionsäuremethylester Hydrochlorid
   3-(4'-Chlor-biphenyl-4-yl)-3-{2-[5-(pyridin-2-ylamino)-pentanoylamino]-acetylamino}-propionsäuremethylester;
mit 3-(2-Amino-acetylamino)-3-(3'-chlor-biphenyl-4-yl)-propionsäuremethylester Hydrochlorid
   3-(3'-Chlor-biphenyl-4-yl)-3-{2-[5-(pyridin-2-ylamino)-pentanoylamino]-acetylamino}-propionsäuremethylester;
mit 3-(2-Amino-acetylamino)-3-(4'-methyl-biphenyl-4-yl)-propionsäuremethylester Hydrochlorid
   3-(4'-Methyl-biphenyl-4-yl)-3-{2-[5-(pyridin-2-ylamino)-pentanoylamino]-acetylamino}-propionsäuremethylester;
mit 3-(2-Amino-acetylamino)-3-[4'-(trifluormethyl)-biphenyl-4-yl]-propionsäuremethylester Hydrochlorid
   3-[4'-(Trifluormethyl)-biphenyl-4-yl]-3-{2-[5-(pyridin-2-ylamino)-pentanoylamino]-acetylamino}-propionsäuremethylester;
mit 3-(2-Amino-acetylamino)-3-(3'-chlor-4'-fluor-biphenyl-4-yl)-propionsäuremethylester Hydrochlorid
   3-(3'-Chlor-4'-fluor-biphenyl-4-yl)-3-{2-[5-(pyridin-2-ylamino)-pentanoylamino]-acetylamino}-propionsäuremethylester;
mit 3-(2-Amino-acetylamino)-3-(4-naphthalin-1-yl-phenyl)-propionsäuremethylester Hydrochlorid
   3-(4-Naphthalin-1-yl-phenyl)-3-{2-[5-(pyridin-2-ylamino)-pentanoylamino]-acetylamino}-propionsäuremethylester;
mit 3-(2-Amino-acetylamino)-3-(4-naphthalin-2-yl-phenyl)-propionsäuremethylester Hydrochlorid
   3-(4-Naphthalin-2-yl-phenyl)-3-{2-[5-(pyridin-2-ylamino)-pentanoylamino]-acetylamino}-propionsäuremethylester oder
mit 3-(2-Amino-acetylamino)-3-(4'-fluor-2-hydroxy-biphenyl-4-yl)-propionsäuremethylester Hydrochlorid
   3-(4'-Fluor-2-hydroxy-biphenyl-4-yl)-3-{2-[5-(pyridin-2-ylamino)-pentanoylamino]-acetylarnino}-propionsäuremethylester.

### Beispiel 19:

Nach Verseifung der Methylester der Verbindungen von Beispiel 18 analog zu Beispiel 2 (4) erhält man
3-(4'-Fluor-biphenyl-4-yl)-3-{2-[5-(pyridin-2-ylamino)-pentanoylamino]-acetylamino}-propionsäure,
3-(4'-Fluor-biphenyl-4-yl)-3-{2-[5-(pyridin-2-ylamino)-pentanoylamino]-acetylamino}-propionsäure Natriumsalz,
3-(4'-Fluor-biphenyl-4-yl)-3-{2-[5-(pyridin-2-ylamino)-pentanoylamino]-acetylamino}-propionsäure Trifluoracetat, RT 24.66 min, FAB-MS (M+H)⁺ 493;

3-(4'-Fluor-biphenyl-3-yl)-3-{2-[5-(pyridin-2-ylamino)-pentanoylamino]-acetylamino}-propionsäure,
3-(4'-Fluor-biphenyl-3-yl)-3-{2-[5-(pyridin-2-ylamino)-pentanoylamino]-acetylamino}-propionsäure Natriumsalz,
3-(4'-Fluor-biphenyl-3-yl)-3-{2-[5-(pyridin-2-ylamino)-pentanoylamino]-acetylamino}-propionsäure Trifluoracetat, RT 25.36 min, FAB-MS (M+H)⁺ 493;

3-(3'-Fluor-biphenyl-4-yl)-3-{2-[5-(pyridin-2-ylamino)-pentanoylamino]-acetylamino}-propionsäure,
3-(3'-Fluor-biphenyl-4-yl)-3-{2-[5-(pyridin-2-ylamino)-pentanoylamino]-acetylamino}-propionsäure Natriumsalz,
3-(3'-Fluor-biphenyl-4-yl)-3-{2-[5-(pyridin-2-ylamino)-pentanoylamino]-acetylamino}-propionsäure Trifluoracetat, FAB-MS (M+H)⁺ 493;

3-(2'-Fluor-biphenyl-4-yl)-3-{2-[5-(pyridin-2-ylamino)-pentanoylamino]-acetylamino}-propionsäure,
3-(2'-Fluor-biphenyl-4-yl)-3-{2-[5-(pyridin-2-ylamino)-pentanoylamino]-acetylamino}-propionsäure Natriumsalz,
3-(2'-Fluor-biphenyl-4-yl)-3-{2-[5-(pyridin-2-ylamino)-pentanoylamino]-acetylamino}-propionsäure Trifluoracetat, FAB-MS (M+H)⁺ 493;

3-(4'-Chlor-biphenyl-4-yl)-3-{2-[5-(pyridin-2-ylamino)-pentanoylamino]-acetylamino}-propionsäure,
3-(4'-Chlor-biphenyl-4-yl)-3-{2-[5-(pyridin-2-ylamino)-pentanoylamino]-acetylamino}-propionsäure Natriumsalz,
3-(4'-Chlor-biphenyl-4-yl)-3-{2-[5-(pyridin-2-ylamino)-pentanoylamino]-acetylamino}-propionsäure Trifluoracetat, FAB-MS (M+H)⁺ 510;

3-(3'-Chlor-biphenyl-4-yl)-3-{2-[5-(pyridin-2-ylamino)-pentanoylamino]-acetylamino}-propionsäure,
3-(3'-Chlor-biphenyl-4-yl)-3-{2-[5-(pyridin-2-ylamino)-pentanoylamino]-acetylamino}-propionsäure Natriumsalz,
3-(3'-Chlor-biphenyl-4-yl)-3-{2-[5-(pyridin-2-ylamino)-pentanoylamino]-acetylamino}-propionsäure Trifluoracetat, FAB-MS (M+H)⁺ 510;
3-(4'-Methyl-biphenyl-4-yl)-3-{2-[5-(pyridin-2-ylamino)-pentanoylamino]-acetylamino}-propionsäure,
3-(4'-Methyl-biphenyl-4-yl)-3-{2-[5-(pyridin-2-ylamino)-pentanoylamino]-acetylamino}-propionsäure Natriumsalz,
3-(4'-Methyl-biphenyl-4-yl)-3-{2-[5-(pyridin-2-yl'amino)-pentanoylamino]-acetylamino}-propionsäure Trifluoracetat, FAB-MS (M+H)⁺ 489;

3-[4'-(Trifluormethyl)-biphenyl-4-yl]-3-{2-[5-(pyridin-2-ylamino)-pentanoylamino]-acetylamino}-propionsäure,
3-[4'-(Trifluormethyl)-biphenyl-4-yl]-3-{2-[5-(pyridin-2-ylamino)-pentanoylamino]-acetylamino}-propionsäure Natriumsalz,
3-[4'-(Trifluormethyl)-biphenyl-4-yl]-3-{2-[5-(pyridin-2-ylamino)-pentanoylamino]-acetylamino}-propionsäure Trifluoracetat, FAB-MS (M+H)⁺ 543;

3-(3'-Chlor-4'-fluor-biphenyl-4-yl)-3-{2-[5-(pyridin-2-ylamino)-pentanoylamino]-acetylamino}-propionsäure,
3-(3'-Chlor-4'-fluor-biphenyl-4-yl)-3-{2-[5-(pyridin-2-ylamino)-pentanoylamino]-acetylamino}-propionsäure Natriumsalz,
3-(3'-Chlor-4'-fluor-biphenyl-4-yl)-3-{2-[5-(pyridin-2-ylamino)-pentanoylamino]-acetylamino}-propionsäure Trifluoracetat, FAB-MS (M+H)⁺ 527;

3-(4-Naphthalin-1-yl-phenyl)-3-{2-[5-(pyridin-2-ylamino)-pentanoylamino]-acetylamino}-propionsäure,
3-(4-Naphthalin-1-yl-phenyl)-3-{2-[5-(pyridin-2-ylamino)-pentanoylamino]-acetylamino}-propionsäure Natriumsalz,
3-(4-Naphthalin-1-yl-phenyl)-3-{2-[5-(pyridin-2-ylamino)-pentanoylamino]-acetylamino}-propionsäure Trifluoracetat, RT* 24.91 min, FAB-MS (M+H)⁺ 525;

3-(4-Naphthalin-2-yl-phenyl)-3-{2-[5-(pyridin-2-ylamino)-pentanoylamino]-acetylamino}-propionsäure,
3-(4-Naphthalin-2-yl-phenyl)-3-{2-[5-(pyridin-2-ylamino)-pentanoylamino]-acetylamino}-propionsäure Natriumsalz,
3-(4-Naphthalin-2-yl-phenyl)-3-{2-[5-(pyridin-2-ylamino)-pentanoylamino]-acetylamino}-propionsäure Trifluoracetat oder

3-(4'-Fluor-2-hydroxy-biphenyl-4-yl)-3-{2-[5-(pyridin-2-ylamino)-pentanoylamino]-acetylamino}-propionsäure,
3-(4'-Fluor-2-hydroxy-biphenyl-4-yl)-3-{2-[5-(pyridin-2-ylamino)-pentanoylamino]-acetylamino}-propionsäure Natriumsalz,
3-(4'-Fluor-2-hydroxy-biphenyl-4-yl)-3-{2-[5-(pyridin-2-ylamino)-pentanoylamino]-acetylamino}-propionsäure Trifluoracetat.

### Beispiel 20:

Analog zu Beispiel 2 erhält man aus 4-(Pyridin-2-ylamino)-buttersäure "CD"
mit 3-(2-Amino-acetylamino)-3-(4'-fluor biphenyl-4-yl)-propionsäuremethylester Hydrochlorid
   3-(4'-Fluor-biphenyl-4-yl)-3-{2-[4-(pyridin-2-ylamino)-butyrylamino]-acetylamino}-propionsäuremethylester;
mit 3-(2-Amino-acetylamino)-3-(4'-fluor-biphenyl-3-yl)-propionsäuremethylester Hydrochlorid
   3-(4'-Fluor-biphenyl-3-yl)-3-{2-[4-(pyridin-2-ylamino)-butyrylamino]-acetylamino}-propionsäuremethylester;
mit 3-(2-Amino-acetylamino)-3-(4-naphthalin-1-yl-phenyl)-propionsäuremethylester Hydrochlorid
   3-(4-Naphthalin-1-yl-phenyl)-3-{2-[4-(pyridin-2-ylamino)-butyrylamino]-acetylamino}-propionsäuremethylester;
mit 3-(2-Amino-acetylamino)-3-(2-hydroxy-4-naphthalin-1-yl-phenyl)-propionsäuremethylester Hydrochlorid
   3-(4-Naphthalin-1-yl-2-hydroxy-phenyl)-3-{2-[4-(pyridin-2-ylamino)-butyrylamino]-acetylamino}-propionsäuremethylester oder
mit 3-(2-Amino-acetylamino)-3-(2-hydroxy-biphenyl-4-yl)-propionsäuremethyl-ester Hydrochlorid
   3-(2-Hydroxy-biphenyl-4-yl)-3-{2-[4-(pyridin-2-ylamino)-butyrylamino]-acetylamino}-propionsäuremethylester.

### Beispiel 21:

Nach Verseifung der Methylester der Verbindungen von Beispiel 20 analog zu Beispiel 2 (4) erhält man
3-(4'-Fluor-biphenyl-4-yl)-3-{2-[4-(pyridin-2-ylamino)-butyrylamino]-acetylamino}-propionsäure,
3-(4'-Fluor-biphenyl-4-yl)-3-{2-[4-(pyridin-2-ylamino)-butyrylamino]-acetylamino}-propionsäure Natriumsalz,
3-(4'-Fluor-biphenyl-4-yl)-3-{2-[4-(pyridin-2-ylamino)-butyrylamino]-acetylamino}-propionsäure Trifluoracetat, RT 24.53, FAB-MS (M+H)⁺ 479;

3-(4'-Fluor-biphenyl-3-yl)-3-{2-[4-(pyridin-2-ylamino)-butyrylamino]-acetylamino}-propionsäure,
3-(4'-Fluor-biphenyl-3-yl)-3-{2-[4-(pyridin-2-ylamino)-butyrylamino]-acetylamino}-propionsäure Natriumsalz,
3-(4'-Fluor-biphenyl-3-yl)-3-{2-[4-(pyridin-2-ylamino)-butyrylamino]-acetylamino}-propionsäure Trifluoracetat, RT 25.06, FAB-MS (M+H)⁺ 479;

3-(4-Naphthalin-1-yl-phenyl)-3-{2-[4-(pyridin-2-ylamino)-butyrylamino]-acetylamino}-propionsäure,
3-(4-Naphthalin-1-yl-phenyl)-3-{2-[4-(pyridin-2-ylamino)-butyrylamino]-acetylamino}-propionsäure Natriumsalz,
3-(4-Naphthalin-1-yl-phenyl)-3-{2-[4-(pyridin-2-ylamino)-butyrylamino]-acetylamino}-propionsäure Trifluoracetat, RT* 24.80, FAB-MS (M+H)⁺ 511;

3-(4-Naphthalin-1-yl-2-hydroxy-phenyl)-3-{2-[4-(pyridin-2-ylamino)-butyrylamino]-acetylamino}-propionsäure,
3-(4-Naphthalin-1-yl-2-hydroxy-phenyl)-3-{2-[4-(pyridin-2-ylamino)-butyrylamino]-acetylamino}-propionsäure Natriumsalz,
3-(4-Naphthalin-1-yl-2-hydroxy-phenyl)-3-{2-[4-(pyridin-2-ylamino)-butyrylamino]-acetylamino}-propionsäure Trifluoracetat oder

3-(2-Hydroxy-biphenyl-4-yl)-3-{2-[4-(pyridin-2-ylamino)-butyrylamino]-acetylamino}-propionsäure,
3-(2-Hydroxy-biphenyl-4-yl)-3-{2-[4-(pyridin-2-ylamino)-butyrylamino]-acetylamino}-propionsäure Natriumsalz,
3-(2-Hydroxy-biphenyl-4-yl)-3-(2-[4-(pyridin-2-ylamino)-butyrylamino]-acetylamino}-propionsäure Trifluoracetat.

### Beispiel 22:

Analog zu Beispiel 2 erhält man aus 4-(4-Methyl-pyridin-2-ylamino)-buttersäure "AB"
mit 3-(2-Amino-acetylamino)-3-(4'-fluor-biphenyl-3-yl)-propionsäuremethylester Hydrochlorid
   3-(4'-Fluor-biphenyl-3-yl)-3-{2-[4-(4-methyl-pyridin-2-ylamino)-butyrylamino]-acetylamino}-propionsäuremethylester;
mit 3-(2-Amino-acetylamino)-3-(4'-fluor-biphenyl-4-yl)-propionsäuremethylester Hydrochlorid
   3-(4'-Fluor-biphenyl-4-yl)-3-{2-[4-(4-methyl-pyridin-2-ylamino)-butyrylamino]-acetylamino}-propionsäuremethylester;
mit 3-(2-Amino-acetylamino)-3-(4-naphthalin-1-yl-phenyl)-propionsäuremethylester Hydrochlorid
   3-{2-[4-(4-Methyl-pyridin-2-ylamino)-butyrylamino]-acetylamino}-3-(4-naphthalin-1-yl-phenyl)-propionsäuremethylester;
mit 3-(2-Amino-acetylamino)-3-(4-naphthalin-1-yl-2-hydroxy-phenyl)-propionsäuremethylester Hydrochlorid
   3-{2-[4-(4-Methyl-pyridin-2-ylamino)-butyrylamino]-acetylamino}-3-(4-naphthalin-1-yl-2-hydroxy-phenyl)-propionsäuremethylester;
mit 3-(2-Amino-acetylamino)-3-(4-naphthatin-2-yl-phenyl)-propionsäuremethylester Hydrochlorid
   3-{2-[4-(4-Methyl-pyridin-2-ylamino)-butyrylamino]-acetylamino}-3-(4-naphthalin-2-yl-phenyl)-propionsäuremethylester;
mit 3-(2-Amino-acetylamino)-3-(4'-fluor-2'-hydroxy-biphenyl-4-yl)-propionsäuremethylester Hydrochlorid
   3-(4'-Fluor-2'-hydroxy-biphenyl-4-yl)-3-{2-[4-(4-methyl-pyridin-2-ylamino)-butyrylamino]-acetylamino}-propionsäuremethylester;
mit 3-(2-Amino-acetylamino)-3-(4'-fluor-2'-hydroxy-biphenyl-3-yl)-propionsäuremethytester Hydrochlorid
   3-(4'-Fluor-2'-hydroxy-biphenyl-3-yl)-3-{2-[4-(4-methyl-pyridin-2-ylamino)-butyrylamino]-acetylamino}-propionsäuremethylester oder
mit 3-(2-Amino-acetylamino)-3-(2-hydroxy-biphenyl-4-yl)-propionsäuremethylester Hydrochlorid
   3-(2-Hydroxy-biphenyl-4-yl)-3-{2-[4-(4-methyl-pyridin-2-ylamino)-butyrylamino]-acetylamino}-propionsäuremethylester.

### Beispiel 23:

Nach Verseifung der Methylester der Verbindungen von Beispiel 22 analog zu Beispiel 2 (4) erhält man
3-(4'-Fluor-biphenyl-3-yl)-3-{2-[4-(4-methyl-pyridin-2-ylamino)-butyrylamino]-acetylamino}-propionsäure,
3-(4'-Fluor-biphenyl-3-yl)-3-{2-[4-(4-methyl-pyridin-2-ylamino)-butyrylamino]-acetylamino}-propionsäure Natriumsalz,
3-(4'-Fluor-biphenyl-3-yl)-3-{2-[4-(4-methyl-pyridin-2-ylamino)-butyrylamino]-acetylamino}-propionsäure Trifluoracetat, RT 26.32 min, FAB-MS (M+H)⁺ 493;

3-(4'-Fluor-biphenyl-4-yl)-3-{2-[4-(4-methyl-pyridin-2-ylamino)-butyrylamino]-acetylamino}-propionsäure,
3-(4'-Fluor-biphenyl-4-yl)-3-{2-[4-(4-methyl-pyridin-2-ylamino)-butyrylamino]-acetylamino}-propionsäure Natriumsalz,
3-(4'-Fluor-biphenyl-4-yl)-3-{2-[4-(4-methyl-pyridin-2-ylamino)-butyrylamino]-acetylamino}-propionsäure Trifluoracetat;

3-{2-[4-(4-Methyl-pyridin-2-ylamino)-butyrylamino]-acetylamino}-3-(4-naphthalin-1-yl-phenyl)-propionsäure.
3-{2-[4-(4-Methyl-pyridin-2-ylamino)-butyrylamino]-acetylamino}-3-(4-naphthalin-1-yl-phenyl)-propionsäure Natriumsalz,
3-{2-[4-(4-Methyl-pyridin-2-ylamino)-buiyrylamino]-acetylamino}-3-(4-naphthalin-1-yl-phenyl)-propionsäure Trifluoracetat, RT* 25.44 min, FAB-MS (M+H)⁺ 525;

3-{2-[4-(4-Methyl-pyridin-2-ylamino)-butyrylamino]-acetylamino}-3-(4-naphthalin-1-yl-2-hydroxy-phenyl)-propionsäure,
3-{2-[4-(4-Methyl-pyridin-2-ylamino)-butyrylamino]-acetylamino}-3-(4-naphthalin-1-yl-2-hydroxy-phenyl)-propionsäure Natriumsalz,
3-{2-[4-(4-Methyl-pyridin-2-ylamino)-butyrylamino]-acetylamino}-3-(4-naphthalin-1-yl-2-hydroxyphenyl)-propionsäure Trifluoracetat;

3-{2-[4-(4-Methyl-pyridin-2-ylamino)-butyrylamino]-acetylamino}-3-(4-naphthalin-2-yl-phenyl)-propionsäure,
3-{2-[4-(4-Methyl-pyridin-2-ylamino)-butyrylamino]-acetylamino}-3-(4-naphthalin-2-yl-phenyl)-propionsäure Natriumsalz,
3-{2-[4-(4-Methyl-pyridin-2-ylamino)-butyrylamino]-acetylamino}-3-(4-naphthalin-2-yl-phenyl)-propionsäure Trifluoracetat;

3-(4'-Fluor-2'-hydroxy-biphenyl-4-yl)-3-{2-[4-(4-methyl-pyridin-2-ylamino)-butyrylamino]-acetylamino}-propionsäure,
3-{4'-Fluor-2'-hydroxy-biphenyl-4-yl)-3-{2-[4-(4-methyl-pyridin-2-ylamino)-butyrylamino]-acetylamino}-propionsäure Natriumsalz,
3-(4'-Fluor-2'-hydroxy-biphenyl-4-yl)-3-{2-[4-(4-methyl-pyridin-2-ylamino)-butyrylamino]-acetylamino}-propionsäure Trifluoracetat;

3-(4'-Fluor-2'-hydroxy-biphenyl-3-yl)-3-{2-[4-(4-methyl-pyridin-2-ylamino)-butyrylamino]-acetylamino}-propionsäure,
3-(4'-Fluor-2'-hydroxy-biphenyl-3-yl)-3-{2-[4-(4-methyl-pyridin-2-ylamino)-butyrylamino]-acetylamino}-propionsäure Natriumsalz,
3-(4'-Fluor-2'-hydroxy-biphenyl-3-yl)-3-{2-[4-(4-methyl-pyridin-2-ylamino)-butyrylamino]-acetylamino}-propionsäure Trifluoracetat oder

3-(2-Hydroxy-biphenyl-3-yl)-3-{2-[4-(4-methyl-pyridin-2-ylamino)-butyrylamino]-acetylamino}-propionsäure,
3-(2-Hydtoxy-biphenyl-3-yl)-3-{2-[4-(4-methyl-pyridin-2-ylamino)-butyrylamino]-acetylamino}-propionsäure Natriumsalz,
3-(2-Hydroxy-biphenyl-3-yl)-3-{2-[4-(4-methyl-pyridin-2-ylamino)-butyrylamino]-acetylamino}-propionsäure Trifluoracetat.

### Beispiel 26: Prodrugs

1. Die Veresterung von 3-(4-Chlor-3-nitro-phenyl)-3-{2-[5-(4-methyl-pyridin-2-ylamino)-pentanoylamino]-acetylamino}-propionsäure durch Aktivierung mit Thionylchlorid und Reaktion mit Ethanol unter Standardbedingungen erzeugt 3-(4-Chlor-3-nitro-phenyl)-3-{2-[5-(4-methyl-pyridin-2-ylamino)-pentanoylamino]-acetylamino}-propionsäureethylester Hydrochlorid, RT 27.95, FAB-MS (M+H)⁺ 520.

Analog entsteht durch Veresterung und anschließender präparativer HPLC
von 3-(4'-Fluor-biphenyl-3-yl)-3-{2-[5-(pyridin-2-ylamino)-pentanoylamino]-acetytamino}-propionsäure
   3-(4'-Fluor-biphenyl-3-yl)-3-{2-[5-(pyridin-2-ylamino)-pentanoylamino]-acetylamino}-propionsäureethylester Trifluoracetat, RT 30.26, FAB-MS (M+H)⁺ 521.

### Beispiel 27: Assays

Die angiogenen Blutgefäße eines Tumors zeigen auffällig das αvβ3-Integrin und können so durch αvβ3-spezifische Inhibitoren gezielt aufgespürt werden.
Es konnten mit Hilfe eines Analyseverfahrens Zelllinien identifiziert werden, die aus humanen Tumoren gewonnen werden konnten und die das Integrin αvβ6 aber nicht das Integrin αvβ3 präsentieren, beispielsweise Detroit 562, HT-29 und UCLA-P3, oder die beide Integrine αvβ3 und αvβ6 präsentieren, beispielsweise Calu-3 und Capan-2. (Analyseverfahren: immunoprecipitation and fluorescence-activated cell sorting analysis). Diese identifizierten Zelllinien wachsen in immungeschwächten Nagetieren, z.B. in nu/nu Mäusen, als subkutane Tumoren.

Inhibitoren des αvβ3-Integrinrezeptors blockieren das Tumorwachstum in der Art, wie bereits beschrieben, daß die in den Tumor einwachsenden Blutgefäße apoptotischen Signalen ausgesetzt sind und durch den programmierten Zelltod (Apoptose) sterben. (Lit: P.C. Brooks, Eur. J. Cancer 1996, 32A, 2423-2429, P.C. Brooks et al., Cell 1994, 79, 1157-1164 oder S. Stomblad et al., J. Clin. Invest 1996, 98, 426-433.)

Die αvβ6-Integrininhibitoren beeinträchtigen direkt die Tumorentwicklung. Der synergistische Effekt der erfindungsgemäßen kombinierten Therapie wird durch die folgende Versuchsreihe analog zu den Testsystemen von Mitjans et al., J. Cell. Sci. 1995, 108, 2825-2838 dokumentiert: αvβ6-exprimierende Tumorzellen werden subkutan z.B. in nu/nu Mäusen implantiert. Analog zur M21-Zelllinie von Mitjans et al. wird das Wachstum dieser Tumorzellen in den Mäusen in Abhängigkeit der Integrininhibitoren beobachtet.

Nach der Implantation der Tumorzellen werden die so präparierten Mäuse separiert und in jeweils Gruppen von 10 Mäusen aufgeteilt. Die Mäuse werden täglich erfindungsgemäß durch eine intraperitoneale Injektion mit den entsprechenden Integrininhibitoren behandelt und das Wachstum der Tumoren beobachtet. Die Kontrollgruppe erhält Injektionen von sterilem pyrogenfreier Salzlösung. Die Tumorgröße wird zwei Mal in der Woche gemessen und das entsprechende Tumorvolumen berechnet.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 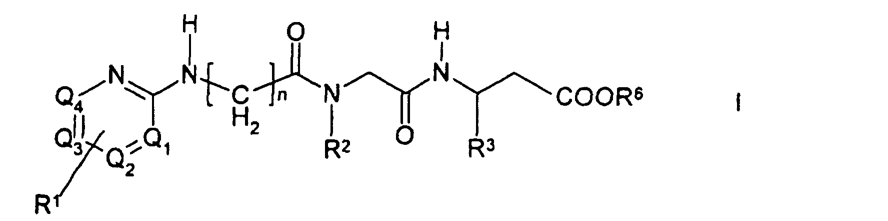 worin
Q₁, Q₂, Q₃ oder Q₄ jeweils unabhängig voneinander CH oder N,
R¹ H, A, Ar, Hal, OH, OA, CF₃ oder OCF₃,
R² H oder A,
R³ 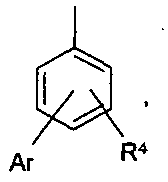
R⁴ H, A, Hal, OH, OA, CF₃, OCF₃, CN, NH₂, NHA, NA₂ oder NH-C(O)A,
R⁶ H, A, -(CH₂)ₘ-OH, -(CH₂)ₘ-O-C(O)A oder -(CH₂)ₘ-Ar,
A Alkyl mit 1 bis 6 C-Atomen,
Ar unsubstituiertes oder ein-, zwei- oder dreifach durch A, CF₃, OH, OA, OCF₃, CN, NO₂ oder Hal substituiertes Aryl, wobei Aryl Phenyl, Naphthyl, Anthryl oder Biphenylyl bedeutet,
Hal F, Cl, Br oder I,
n 2, 3, 4, 5 oder 6,
m 1, 2, 3 oder 4,
bedeutet
sowie ihre physiologisch unbedenklichen Salze und Solvate.

2. Verbindungen nach Anspruch 1
(a) 3-{2-[4-(4-Methyl-pyridin-2-ylamino)-butyrylamino]-acetylamino}-3-(biphenyl-4-yl)-propionsäure,
(b) 3-{2-[4-(4-Methyl-pyridin-2-ylamino)-butyrylamino]-acetylamino}-3-(4-biphenylyl)-propionsäure,
(c) 3-Biphenyl-4-yl-3-{2-[5-(4-methyl-pyridin-2-ylamino)-pentanoylamino]-acetylamino}-propionsäure,
(d) 3-Biphenyl-4-yl-3-{2-[5-(Pyridin-2-ylamino)-pentanoylamino]-acetylamino}-propionsäure,
(e) 3-Biphenyl-4-yl-3-{2-[4-(Pyridin-2-ylamino)-butyrylamino]-acetylamino}-propionsäure,
(f) 3-Biphenyl-4-yl-3-{2-[5-(Pyridin-2-ylamino)-pentanoylamino]-acetylamino}-propionsäuremethylester,
(g) 3-(4'-Fluor-biphenyl-4-yl)-3-{2-[4-(pyridin-2-ylamino)-butyrylamino]-acetylamino}-propionsäure,
(h) 3-(4'-Fluor-biphenyl-4-yl)'-3-{2-[5-(pyridin-2-ylamino)-pentanoylamino]-acetylamino}-propionsäure,
(i) 3-(4-Naphthalin-1-yl-phenyl)-3-{2-[4-(pyridin-2-ylamino)-butyrylamino]-acetylamino}-propionsäure,
(j) 3-{2-[4-(4-Methyl-pyridin-2-ylamino)-butyrylamino]-acetylamino}-3-(4-naphthalin-1-yl-phenyl)-propionsäure,
(k) 3-(4-Naphthalin-1-yl-phenyl)-3-{2-[5-(pyridin-2-ylamino)-pentanoylamino]-acetylamino}-propionsäure,
(l) 3-(4-Naphthalen-2-yl-phenyl)-3-{2-{5-(4-methyl-pyridin-2-ylamino)-pentanoylamino]-acetylamino}-propionsäure.

3. Verfahren zur Herstellung der Verbindungen der Formel I nach Anspruch 1 oder 2 sowie ihrer Salze und Solvate **dadurch gekennzeichnet, daß** man
(a) eine Verbindung der Formel II 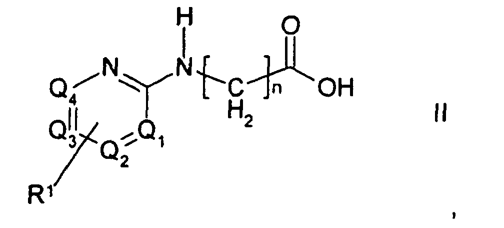 worin Q₁, Q₂, Q₃, Q₄, R¹ und n die in Anspruch 1 angegebenen Bedeutungen haben,
mit einer Verbindung der Formel III 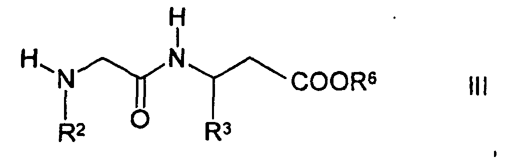 worin R², R³, R⁴, R⁵ und R⁶ die in Anspruch 1 angegebenen Bedeutungen haben
umsetzt
und gegebenenfalls den Rest R⁶ ≠ H in den Rest R⁶ = H umwandelt,
oder
(b) eine Verbindung der Formel IV 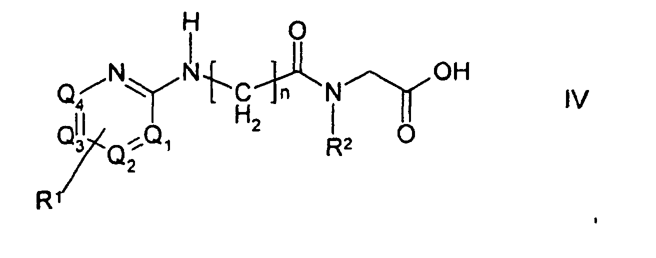 worin Q₁, Q₂, Q₃, Q₄, R¹, R² und n die in Anspruch 1 angegebenen Bedeutungen haben,
mit einer Verbindung der Formel V 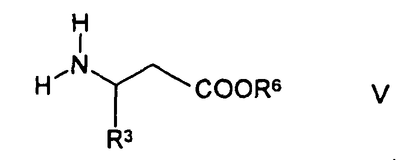 worin R³, R⁴, R⁵ und R⁶ die in Anspruch 1 angegebenen Bedeutungen haben
umsetzt
und gegebenenfalls den Rest R⁶ ≠ H in den Rest R⁶ = H umwandelt,
oder
(c) in einer Verbindung der Formel I einen oder mehrere Reste R¹, R², R³, R⁴ und/oder R⁵ in einen oder mehrere Reste R¹, R², R³, R⁴ und/oder R⁵ umwandelt,
indem man beispielsweise
i) eine Hydroxygruppe alkyliert,
ii) eine Estergruppe zu einer Carboxygruppe hydrolysiert,
iii) eine Carboxygruppe verestert,
iv) eine Aminogruppe alkyliert oder
v) eine Aminogruppe acyliert,
und/oder
eine basische oder saure Verbindung der Formel I durch Behandeln mit einer Säure oder Base in eines ihrer Salze oder Solvate umwandelt.

4. Verbindungen der Formel I nach Anspruch 1 oder 2 und ihre physiologisch unbedenklichen Salze oder Solvate als Arzneimittetwirkstoffe.

5. Verbindungen der Formel I nach Anspruch 1 oder 2 und ihre physiologisch unbedenklichen Salze oder Solvate als Integrininhibitoren.

6. Pharmazeutische Zubereitung **gekennzeichnet durch** einen Gehalt an mindestens einer Verbindung der Formel I nach Anspruch 1 oder 2 und/oder einem ihrer physiologisch unbedenklichen Salze oder Solvate.

7. Verwendung von Verbindungen der Formel I nach Anspruch 1 oder 2 und/oder ihre physiologisch unbedenklichen Salze oder Solvate zur Herstellung eines Arzneimittels.

8. Verwendung von Verbindungen der Formel I nach Anspruch 1 oder 2 und/oder ihrer physiologisch unbedenklichen Salze oder Solvate zur Herstellung eines Arzneimittels zur Bekämpfung von Thrombosen, Herzinfarkt, koronaren Herzerkrankungen, Arteriosklerose, Entzündungen, Tumoren, Osteoporose, Infektionen und Restenose nach Angioplastie.

9. Verwendung von Verbindungen der Formel I nach Anspruch 1 oder 2 und/oder ihre physiologisch unbedenklichen Salze oder Solvate zur Herstellung eines Arzneimittels zur Bekämpfung von pathologischen Vorgängen, die durch Angiogenese unterhalten oder propagiert werden.

10. Verwendung von 3-(Biphenyl-4-yl)-3-{2-[5-(pyridin-2-ylamino)pentanoylamino]-acetylamino}-propionsäure Trifluoracetat zur Herstellung eines Arzneimittels zur Bekämpfung von Krankheiten, die mit Krebs assoziiert sind, wie Metastasen von soliden Tumoren, Angiofibromatose, retrolentale Fibroplasie, Hemangioma oder Kaposi Sarkoma.

## Claims

1. Compounds of the formula I 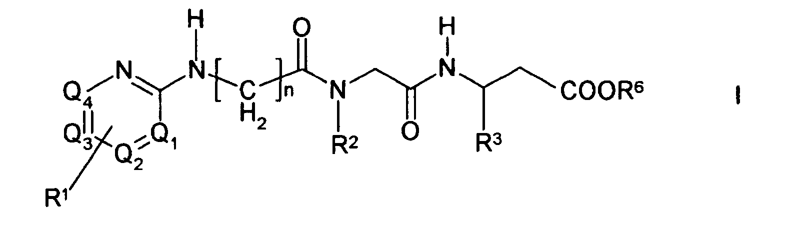 in which
Q₁, Q₂, Q₃ or Q₄ each, independently of one another, denote CH or N,
R' denotes H, A, Ar, Hal, OH, OA, CF₃ or OCF₃,
R² denotes H or A,
R³ denotes 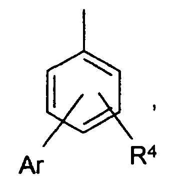
R⁴ denotes H, A, Hal, OH, OA, CF₃, OCF₃, CN, NH₂, NHA, NA₂ or NH-C(O)A,
R⁶ denotes H, A, -(CH₂)ₘ-OH, -(CH₂)ₘ-O-C(O)A or -(CH₂)ₘ-Ar,
A denotes alkyl having 1 to 6 C atoms,
Ar denotes aryl which is unsubstituted or mono-, di- or trisubstituted by A, CF₃, OH, OA, OCF₃, CN, NO₂ or Hal, where aryl denotes phenyl, naphthyl, anthryl or biphenylyl,
Hal denotes F, Cl, Br or I,
n denotes 2, 3, 4, 5 or 6,
m denotes 1, 2, 3 or 4,
and physiologically acceptable salts and solvates thereof.

2. Compounds according to Claim 1
(a) 3-{2-[4-(4-methylpyridin-2-ylamino)butyrylamino]acetylamino}-3-(biphenyl-4-yl)propionic acid,
(b) 3-{2-[4-(4-methylpyridin-2-ylamino)butyrylamino]acetylamino}-3-(4-biphenylyl)propionic acid,
(c) 3-biphenyl-4-yl-3-{2-[5-(4-methylpyridin-2-ylamino)pentanoylamino]-acetylamino}propionic acid,
(d) 3-biphenyl-4-yl-3-{2-[5-(pyridin-2-ylamino)pentanoylamino]acetylamino}propionic acid,
(e) 3-biphenyl-4-yl-3-{2-[4-(pyridin-2-ylamino)butyrylamino]acetylamino}propionic acid,
(f) methyl 3-biphenyl-4-yl-3-{2-[5-(pyridin-2-ylamino)pentanoylamino]-acetylamino}propionate,
(g) 3-(4'-fluorobiphenyl-4-yl)-3-{2-[4-(pyridin-2-ylamino)butyrylamino]-acetylamino}propionic acid,
(h) 3-(4'-fluorobiphenyl-4-yl)-3-{2-[5-(pyridin-2-ylamino)pentanoylamino]acetylamino}propionic acid,
(i) 3-(4-naphthalen-1-ylphenyl)-3-{2-[4-(pyridin-2-ylamino)butyrylamino]acetylamino}propionic acid,
(j) 3-{2-[4-(4-methylpyridin-2-ylamino)butyrylamino]acetylamino}-3-(4-naphthalen-1-ylphenyl)propionic acid,
(k) 3-(4-naphthalen-1-ylphenyl)-3-{2-[5-(pyridin-2-ylamino)pentanoylamino]acetylamino}propionic acid,
(l) 3-(4-naphthalen-2-ylphenyl)-3-{2-[5-(4-methylpyridin-2-ylamino)-pentanoylamino]acetylamino}propionic acid.

3. Process for the preparation of the compounds of the formula I according to Claim 1 or 2 and salts and solvates thereof, **characterised in that**
(a) a compound of the formula II 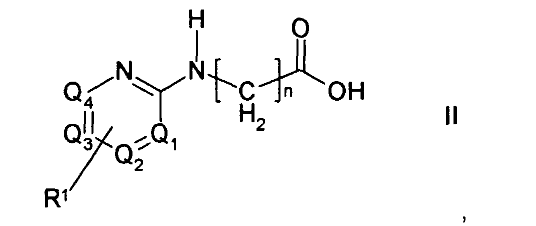 in which Q₁, Q₂, Q₃, Q₄, R¹ and n have the meanings indicated in Claim 1,
is reacted with a compound of the formula III 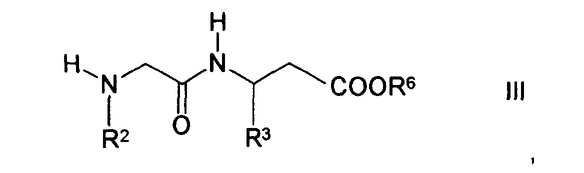 in which R², R³, R⁴, R⁵ and R⁶ have the meanings indicated in Claim 1,
and the radical R⁶ ≠ H is optionally converted into the radical R⁶ = H,
or
(b) a compound of the formula IV 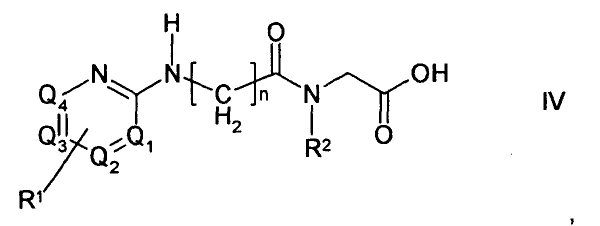 in which Q₁, Q₂, Q₃, Q₄, R¹, R² and n have the meanings indicated in Claim 1,
is reacted with a compound of the formula V 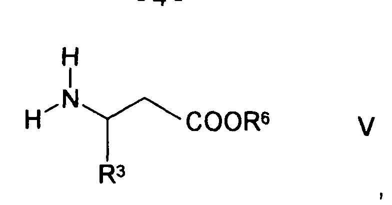 in which R³, R⁴, R⁵ and R⁶ have the meanings indicated in Claim 1, and the radical R⁶ ≠ H is optionally converted into the radical R⁶ = H,
or
(c) one or more radicals R¹, R², R³, R⁴ and/or R⁵ in a compound of the formula I are converted into one or more radicals R¹, R², R³, R⁴ and/or R⁵
by, for example,
i) alkylating a hydroxyl group,
ii) hydrolysing an ester group to a carboxyl group,
iii) esterifying a carboxyl group,
iv) alkylating an amino group or
v) acylating an amino group,
and/or
a basic or acidic compound of the formula I is converted into one of its salts or solvates by treatment with an acid or base.

4. Compounds of the formula I according to Claim 1 or 2 and physiologically acceptable salts or solvates thereof as medicament active ingredients.

5. Compounds of the formula I according to Claim 1 or 2 and physiologically acceptable salts or solvates there as integrin inhibitors.

6. Pharmaceutical composition **characterised by** a content of at least one compound of the formula I according to Claim 1 or 2 and/or one of its physiologically acceptable salts or solvates.

7. Use of compounds of the formula I according to Claim 1 or 2 and/or physiologically acceptable salts or solvates thereof for the preparation of a medicament.

8. Use of compounds of the formula I according to Claim 1 or 2 and/or physiologically acceptable salts or solvates thereof for the preparation of a medicament for combating thromboses, cardiac infarction, coronary heart diseases, arteriosclerosis, inflammation, tumours, osteoporosis, infections and restenosis after angioplasty.

9. Use of compounds of the formula I according to Claim 1 or 2 and/or physiologically acceptable salts or solvates thereof for the preparation of a medicament for combating pathological processes that are supported or propagated by angiogenesis.

10. Use of 3-(biphenyl-4-yl)-3-{2-[5-(pyridin-2-ylamino)pentanoylamino]-acetylamino}propionic acid trifluoroacetate for the preparation of a medicament for combating diseases associated with cancer, such as metastases of solid tumours, angiofibromatosis, retrolental fibroplasia, haemangioma or Kaposi's sarcoma.

## Revendications

1. Composés de la formule I 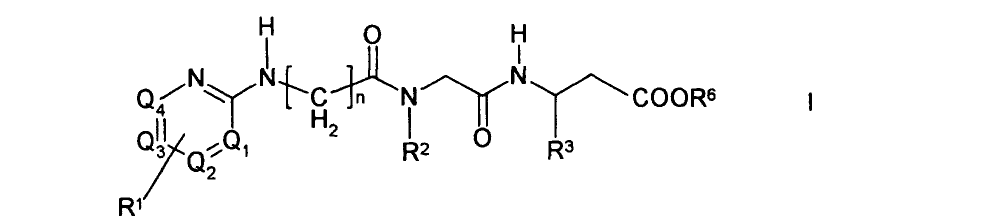 dans laquelle
Q₁, Q₂, Q₃ ou Q₄ chacun, indépendamment les uns des autres, représentent CH ou N,
R¹ représente H, A, Ar, Hal, OH, OA, CF₃ ou OCF₃,
R² représente H ou A,
R³ denotes 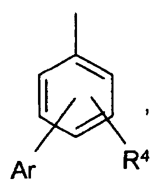
R⁴ représente H, A, Hal, OH, OA, CF₃, OCF₃, CN, NH₂, NHA, NA₂ ou NH-C(O)A,
R⁶ représente H, A, -(CH₂)ₘ-OH, -(CH₂)ₘ-O-C(O)A ou -(CH₂)ₘ-Ar,
A représente alkyle ayant 1 à 6 atomes de C,
Ar représente aryle qui est non substitué ou mono-, di- ou trisubstitué par A, CF₃, OH, OA, OCF₃, CN, NO₂ ou Hal, où aryle représente phényle, naphthyle, anthryle ou biphénylyle,
Hal représente F, Cl, Br ou I,
n représente 2, 3, 4, 5 ou 6,
m représente 1, 2, 3 ou 4,
et leurs sels et solvates physiologiquement acceptables.

2. Composés selon la revendication 1
(a) acide 3-{2-[4-(4-méthylpyridine-2-ylamino)butyrylamino]acétylamino}-3-(biphényl-4-yl)propionique,
(b) acide 3-{2-[4-(4-méthylpyridine-2-ylamino)butyrylamino]acétylamino}-3-(4-biphénylyl)propionique,
(c) acide 3-biphényl-4-yl-3-(2-[5-(4-méthylpyridine-2-ylamino)-pentanoylamino]acétylamino}propionique,
(d) acide 3-biphényl-4-yl-3-{2-[5-(pyridin-2-ylamino)pentanoylamino]-acétylamino}propionique,
(e) acide 3-biphényl-4-yl-3-{2-[4-(pyridine-2-ylamino)butyrylamino]-acétylamino}propionique,
(f) méthyl 3-biphényl-4-yl-3-{2-[5-(pyridine-2-ylamino)pentanoylamino]-acétylamino}propionate,
(g) acide 3-(4'-fluorobiphényl-4-yl)-3-{2-[4-(pyridine-2-ylamino)butyrylamino]acétylamino}propionique,
(h) acide 3-(4'-fluorobiphényl-4-yl)-3-{2-[5-(pyridine-2-ylamino)-pentanoylamino]acétylamino}propionique,
(i) acide 3-(4-naphthalène-1-ylphényl)-3-{2-[4-(pyridine-2-ylamino)-butyrylamino]acétylamino}propionique,
(j) acide 3-{2-[4-(4-méthylpyridine-2-ylamino)butyrylamino]acétylamino}-3-(4-naphthalène-1-ylphényl)propionique,
(k) acide 3-(4-naphthalène-1-ylphényl)-3-{2-[5-(pyridine-2-ylamino)-pentanoylamino]acétylamino}propionique,
(l) acide 3-(4-naphthalène-2-ylphényl)-3-{2-[5-(4-méthylpyridine-2-yl-amino)pentanoylamino]acétylamino}propionique.

3. Procédé pour la préparation des composés de la formule I selon la revendication 1 ou 2 et leurs sels et solvates, **caractérisé en ce que**
(a) a compound de la formule II 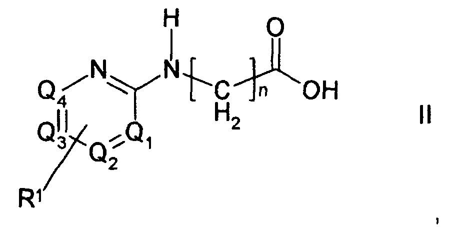 dans laquelle Q₁, Q₂, Q₃, Q₄, R¹ et n ont les significations indiquées dans la revendication 1,
est amené à réagir avec un composé de la formule III 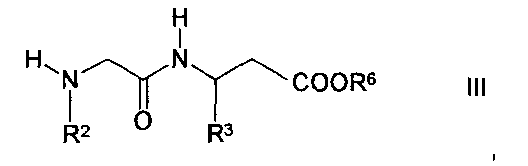 dans laquelle R², R³, R⁴, R⁵ et R⁶ ont les significations indiquées dans la revendication 1,
et le radical R⁶ ≠ H est optionnellement converti selon le radical R⁶ = H,
ou
(b) un composé de la formule IV 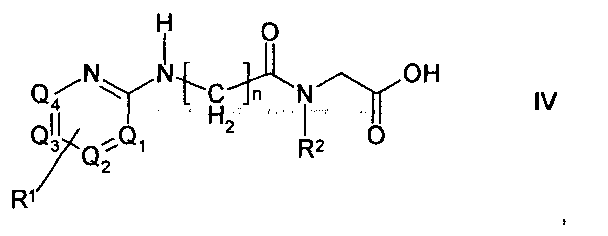 dans laquelle Q₁, Q₂, Q₃, Q₄, R¹, R² et n ont les significations indiquées dans la revendication 1,
est amené à réagir avec un composé de la formule V 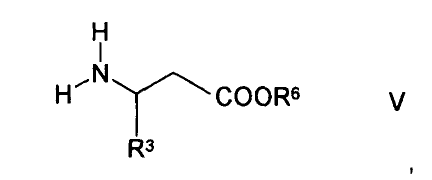 dans laquelle R³, R⁴, R⁵ et R⁶ ont les significations indiquées dans la revendication 1,
et le radical R⁶ ≠ H est optionnellement converti selon le radical R⁶ = H,
ou
(c) un ou plusieurs radicaux R¹, R², R³, R⁴ et/ou R⁵ dans un composé de la formule I sont convertis selon un ou plusieurs radicaux R¹, R², R³, R⁴ et/ou R⁵
par, par exemple,
i) alkylatant un groupe hydroxyle,
ii) hydrolysant un groupe ester selon un groupe corboxyle,
iii) estérifiant un groupe carboxyle,
iv) alkylatant un groupe amino ou
v) acylatant un groupe amino,
et/ou
un composé basique ou acide de la formule I est converti selon l'un de ses sels ou solvates par traitement avec un acide ou une base.

4. Composés de la formule I selon la revendication 1 ou 2 et leurs sels ou solvates physiologiquement acceptables en tant qu'ingrédients actifs de médicaments.

5. Composés de la formule I selon la revendication 1 ou 2 et leurs sels ou solvates physiologiquement acceptables en tant qu'inhibiteurs d'intégrine.

6. Composition pharmaceutique **caractérisée par** une teneur en au moins un composé de la formule I selon la revendication 1 ou 2 et/ou l'un de ses sels ou solvates physiologiquement acceptables.

7. Utilisation de composés de la formule I selon la revendication 1 ou 2 et/ou leurs sels ou solvates physiologiquement acceptables pour la préparation d'un médicament.

8. Utilisation de composés de la formule I selon la revendication 1 ou 2 et/ou leurs sels ou solvates physiologiquement acceptables pour la préparation d'un médicament pour combattre des thromboses, un infarctus cardiaque, des maladies cardiaques coronariennes, des artérioscléroses, des inflammations, des tumeurs, des ostéoporoses, des infections et des resténoses après une angioplastie.

9. Utilisation de composés de la formule I selon la revendication 1 ou 2 et/ou leurs sels ou solvates physiologiquement acceptables pour la préparation d'un médicament pour combattre des processus pathologiques qui sont supportés ou propagés par angionèse.

10. Utilisation de trifluoracétate d'acide 3-(biphényl-4-yl)3-{2-[5-(pyridine-2-ylamino)pentanoylamino]acétylamino}propionique pour la préparation d'un médicament pour combattre des maladies associées au cancer, tel que des métastases de tumeurs solides, une angiofibromatose, une fibroplasie rétrocristallinaire, un hémangiome ou un sarcome de Kaposi.
